(19) Europäisches Patentamt
European Patent Office
Office européen des brevets

(11) EP 2 125 115 B1

(12) **EUROPÄISCHE PATENTSCHRIFT**

(45) Veröffentlichungstag und Bekanntmachung des Hinweises auf die Patenterteilung:
**20.01.2016 Patentblatt 2016/03**

(21) Anmeldenummer: **08707209.6**

(22) Anmeldetag: **23.01.2008**

(51) Int Cl.:
***A61Q 17/04*** *(2006.01)* ***A61K 8/11*** *(2006.01)*
***A61K 8/19*** *(2006.01)* ***A61K 8/26*** *(2006.01)*
***A61K 8/29*** *(2006.01)* ***B65D 81/30*** *(2006.01)*
***C09D 7/12*** *(2006.01)* ***D06M 11/36*** *(2006.01)*

(86) Internationale Anmeldenummer:
**PCT/EP2008/000496**

(87) Internationale Veröffentlichungsnummer:
**WO 2008/092590 (07.08.2008 Gazette 2008/32)**

(54) **PARTIKULÄRES UV-SCHUTZMITTEL**

PARTICULATE UV PROTECTION AGENT

AGENTS PARTICULAIRES DE PROTECTION CONTRE LES UV

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **29.01.2007 DE 102007005186**

(43) Veröffentlichungstag der Anmeldung:
**02.12.2009 Patentblatt 2009/49**

(73) Patentinhaber:
- **Merck Patent GmbH**
  **64293 Darmstadt (DE)**
- **Sachtleben Chemie GmbH**
  **47198 Duisburg (DE)**

(72) Erfinder:
- **PFLUECKER, Frank**
  **64297 Darmstadt (DE)**
- **HIRTHE, Bernd**
  **47918 Tönisvorst (DE)**

(56) Entgegenhaltungen:
**WO-A-2007/141342** **WO-A-2008/015056**
**DE-A1- 19 826 379** **GB-A- 1 162 799**
**GB-A- 1 222 955** **US-A- 5 643 592**
**US-A1- 2005 249 682**

- **G. WAKEFIELD, J. STOTT, J. HOCK: "Sonnenschutzmittel und Kosmetika mit Mangan dotierten Titanoxid-Nanopartikeln" SÖFW-JOURNAL, Bd. 131, 2005, Seiten 60-64, XP002536694**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft partikuläre UV-Schutzmittel nach Anspruch 1, wässrige oder ölige Dispersionen enthaltend diese partikulären UV-Schutzmittel, deren Herstellung und Verwendung. Die vorliegende Erfindung betrifft weiter neue Zusammensetzungen zur topischen Anwendung, die insbesondere zum Lichtschutz der Haut und/oder der Haare gegen UV-Strahlung bestimmt sind (Zusammensetzungen, die im folgenden einfach als Sonnenschutzmittel bezeichnet werden), sowie ihre Verwendung.

Es ist bekannt, dass durch Lichtstrahlung mit einer Wellenlänge im Bereich von 280 bis 400 nm die menschliche Epidermis gebräunt werden kann und dass Strahlung mit einer Wellenlänge im Bereich von 280 bis 320 nm, die unter Bezeichnung UV-B bekannt ist, Erytheme und Hautverbrennungen hervorruft, die der Ausbildung von natürlicher Bräune abträglich sein können. Die UV-B-Strahlung sollte deshalb ausgefiltert werden.

Es ist ferner bekannt, dass UV-A-Strahlung mit einer Wellenlänge im Bereich von 320 bis 400 nm, die die Haut bräunt, eine Veränderung der Haut hervorrufen kann, insbesondere im Falle von empfindlicher Haut oder Haut, die kontinuierlich Sonnenstrahlung ausgesetzt ist. UV-A-Strahlung bewirkt insbesondere einen Verlust der Elastizität der Haut und das Auftreten von Falten, was zu einer vorzeitigen Alterung führt. Sie begünstigt das Auslösen einer Erythembildung oder verstärkt diese Reaktion bei manchen Personen, und sie kann sogar die Ursache für durch Licht ausgelöste toxische oder allergische Reaktionen sein. Es ist daher wünschenswert, auch die UV-A-Strahlung auszufiltern.

In der Kosmetik wurden bis jetzt zahlreiche organische Sonnenschutzfilter angegeben, welche die schädliche UV-A-Strahlung mehr oder weniger selektiv absorbieren können.

Eine diesbezüglich besonders interessante Gruppe von UV-A-Filtern besteht gegenwärtig aus den Dibenzoylmethanderivaten, insbesondere 4,4'-Methoxy-tert.-butyldibenzoylmethan, die ein starkes intrinsisches Absorptionsvermögen aufweisen. Diese Dibenzoylmethanderivate, die derzeit als im UV-A-Bereich wirksame Filter an sich wohlbekannte Produkte sind, sind insbesondere in den französischen Patentanmeldungen FR-A-2 326 405 und FR-A-2 440 933 sowie in der europäischen Patentanmeldung EP-A-0 114 607 beschrieben. Das 4,4'-Methoxy-tert.-butyldibenzoylmethan ist im Übrigen gegenwärtig unter der Handelsbezeichnung Eusolex®9020 von der Fa. Merck im Handel erhältlich.

Diese Dibenzoylmethanderivate können mit einem UV-B-Filter kombiniert werden, um einen vollständigen Schutz über das gesamte Spektrum des Sonnenlichts im UV-Bereich zu erhalten.

Es ist ferner bekannt, dass durch die Zugabe eines anorganischen Pigments und insbesondere eines Pigments von Titandioxid ($TiO_2$), die Lichtschutzeigenschaften der Sonnenschutzmittel, die UV-Filter enthalten, verbessert werden können.

Deshalb ist die Kombination von organischen UV-Filtern, wie z.B. Dibenzoylmethanderivaten und partikulären Metalloxiden auf dem Gebiet der Sonnenschutzmittel sehr geschätzt.

Weiter kann Titandioxid nach Anregung durch UV Strahlung prooxidativ wirken und so zur Bildung von Hydroxylbzw. Peroxid-Radikalen beitragen. Diese Effekte sind insbesondere beim Einsatz von Titandioxid in kosmetischen Sonnenschutzmitteln unerwünscht, da durch solche Radikale die Haut gestresst werden kann.

Es wird ferner beobachtet, dass diese Phänomene im Falle von mikronisiertem $TiO_2$ besonders ausgeprägt sind.

In der WO 99/60994 wird vorgeschlagen Titandioxid mit $Mn^{3+}$-Ionen zu dotieren, um die prooxidativen Eigenschaften zu mildern.

Jetzt wurde überraschend gefunden, dass es möglich ist, die prooxidativen Eigenschaften zu unterbinden, wenn Titandioxide eine Mangan-haltige Beschichtung aufweisen.

Ein erster Gegenstand der vorliegenden Erfindung ist daher ein UV-Schutzmittel, wobei der UV-Schutz im Wesentlichen von einem partikulären Titandioxid ausgeht, dadurch gekennzeichnet, dass das Titandioxid eine Manganhaltige Beschichtung aufweist und dass die Kristallitgröße nach Anspruch 1 gegeben ist.

US 5,643,592 beschreibt partikuläre Additive für Polymere mit einer Oberflächenbehandlung enthaltend eine Verbindung ausgewählt aus der Gruppe von Estern von bifunktionalen $C_6$ bis $C_{40}$ aliphatischen und aromatischen Carbonsäuren und Triestern der Phosphorsäure sowie entsprechende Polymerzusammensetzungen. Als beispielhaftes partikuläres Additiv wird das Handelsprodukt LOCR SM von Sachtleben beschrieben. Laut D1 besteht dieses Handelsprodukt aus einem Titandioxid-Grundkörper, welches 0.01 bis 1 % $SiO_2$, 1 bis 3% $Al_2O_3$, 0.01 bis 1 % Mangan und 0.01 bis 2% Phosphat enthält

DE 198 26 379 beschreibt Korrosionsschutz-Weißpigmente, wobei auf den Titandioxid-Grundkörper $Mn_3(PO_4)_2$ und eine oder mehrere der Substanzen, ausgewählt aus Aluminiumoxiden, -hydroxiden, -phosphaten, - hydrogenphosphaten, -dihydrogenphosphaten und -polyphosphaten aufgefällt sind. DE 198 26 379 beschreibt die als Weißpigmente oder Füllstoffe in Lacken und Farben üblicherweise verwendeten $TiO_2$-Pigmente mit einer Korngröße von 0,1 bis 1,0 $\mu$m.

Die Dokumente GB 1 222 955 und GB 1 162 799 beschreiben Titandioxidpigmente, die mit einer Antimon- und Manganverbindung überzogen sind, als Mattierungsmittel für synthetische Fasern.

WO 2007/141342, ein Dokument, das einen Stand der Technik nach Artikel 54(3) EPÜ darstellt, beschreibt Titandioxid-Pigmente in der Anatas-Modifikation vorliegt und einen Extinktionswert im Bereich von 0,9 bis 1,2 aufweist, das gegebenenfalls mit einer Schicht oder mehreren Schichten anorganischer und/oder organischer Substanzen belegt

ist. Als anorganische Substanzen werden Verbindungen des Aluminiums, des Siliziums, des Zirkons, des Mangans und/oder des Titans verwendet.

WO 2008/015056, ein Dokument, das einen Stand der Technik nach Artikel 54(3) EPÜ darstellt, beschreibt spezielle Oxidpartikel enthaltend Zink und Mangan.

Die Mangan-haltige Beschichtung besteht aus Manganhaltigen Verbindungen, die im Sinne der Erfindung besonders bevorzugt Manganoxide, beispielsweise Mangandioxid und/oder Manganhydroxide sind.

In einer besonders bevorzugten Erfindungsvariante ist das UV-Schutzmittel erhältlich durch hydrothermale Behandlung des partikulären Titandioxids und anschließendes Aufbringen mindestens einer Beschichtung.

Als Hydrothermalbehandlung wird das Erhitzen einer wässrigen Lösung, bzw. Suspension oder Dispersion in einem geschlossenen Behälter, gegebenenfalls unter Druck, bezeichnet (vgl. auch Ullmanns Enzyklopädie der Technischen Chemie, 4. Auflage, 1978, Band 15, S.117 ff: K.Recker, Einkristallzüchtung).

Unter einem partikulären UV-Schutzmittel wird im Sinne einer Erfindungsvariante vorzugsweise ein partikuläres Titandioxid mit Manganhaltiger Siliciumdioxid- oder Aluminiumoxid-Beschichtung verstanden.

Die Kristallitgröße des partikulären Titandioxids in dem partikulären UV-Schutzmittel bestimmt nach der Scherrer-Methode liegt im Bereich von 8 bis 50 nm und insbesondere bevorzugt unterhalb von 25 nm. Die im Transmissions elektronenmikroskop ermittelbaren Abmessungen des partikulären Metalloxides liegen bei einer Länge von 20 bis 60 nm und einer Breite von 8 bis 30 nm. .

Weitere partikuläre Metalloxide sind neben Titandioxid, Eisenoxide, Zinkoxid oder auch Ceroxide. Titandioxid kann in Form von Rutil oder Anatas oder in amorpher Form, vorzugsweise aber in Form von Rutil und/oder Anatas, vorliegen. Dabei sind die Primärpartikel insbesondere bei Anatas vorzugsweise rund, während Rutil-Primärpartikel häufig in Nadel- oder Spindelform bis hin zu Ovalen ("eiförmig") auftreten. Es können erfindungsgemäß jedoch auch runde Rutil-Primärpartikel eingesetzt werden. Weiter können in einer Erfindungsvariante Mischungen von Rutil und Anatas eingesetzt werden.

Partikuläres Titandioxid, welches sich zur erfindungsgemäßen Beschichtung eignet, kann nach verschiedenen Methoden erhalten werden, die dem Fachmann wohl bekannt sind. Beispielsweise kann die Herstellung über einen pyrogenen Prozess (wie z.B. mittels Flammenpyrolyse), mittels eines Sol-Gel Prozesses, eines Plasmaprozesses durch einen Hydrothermalprozess oder durch eine Kombination der verschiedenen Prozessvarianten erfolgen.

Dabei kann das Titandioxid auch dotiert sein. Unter einer Dotierung wird dabei im Sinne der vorliegenden Erfindung die Anwesenheit entsprechender Ionen in geringen Mengen als Störstellen im Kristallgitter des Titandioxids verstanden. Bevorzugte Dotierungen sind dabei solche mit Eisen- oder Cer-Ionen. Ganz besonders bevorzugt ist eine Dotierung des Titandioxids mit Eisen-Ionen.

In einer Erfindungsvariante kann es auch bevorzugt sein, wenn das Titandioxid mit Mangan-Ionen dotiert ist.

In einer weiteren Erfindungsvariante ist es jedoch bevorzugt, wenn das Titandioxid nicht mit Mangan-Ionen dotiert ist. Es kann erfindungsgemäß sogar bevorzugt sein, wenn das Titandioxid gar nicht dotiert ist.

Die Mangan-haltige Beschichtung soll das partikuläre Titandioxid möglichst vollständig bedecken. Da die Beschichtung als UV-Filter jedoch inert ist, ist es erfindungsgemäß bevorzugt, wenn die Menge an Beschichtung niedrig gehalten wird. Es hat sich gezeigt, dass es vorteilhaft ist, wenn die gesamte Beschichtung des Titandioxids bezogen auf das gesamte partikuläre UV-Schutzmittel 5 Gew.% bis 50 Gew.%, vorzugsweise 8 Gew.% bis 30 Gew.% und insbesondere bevorzugt 12 Gew.% bis 20 Gew.% beträgt.

Der Anteil der Manganhaltigen Schicht bezogen auf das gesamte partikuläre UV-Schutzmittel beträgt 0.1 Gew.% bis 1 Gew.%, vorzugsweise 0.2 Gew.% bis 0.7 Gew.% und insbesondere bevorzugt 0.2 Gew.% oder 0.5 Gew.%.

Dabei kann es bevorzugt sein, wenn der Titandioxid Grundkörper eine erste Beschichtung im Wesentlichen bestehend aus Mangan-Verbindungen trägt und eine zweite Beschichtung im Wesentlichen bestehend aus Aluminium- und/oder Siliciumverbindungen trägt. In einer anderen Erfindungsvariante trägt der Titandioxid Grundkörper eine erste Beschichtung im Wesentlichen bestehend aus Aluminium und/oder Siliciumverbindungen und eine zweite Beschichtung im Wesentlichen bestehend aus Mangan-Verbindungen. Erfindungsgemäß kann es ebenfalls beorzugt sein, wenn der Titandioxid-Grundkörper eine Beschichtung im Wesentlichen bestehend oder bestehend aus einem Gemisch von Mangan-Verbindungen mit Aluminium- und/oder Siliciumverbindungen trägt. In einer bevorzugten Erfindungsvariante trägt der Titandioxid-Grundkörper eine erste Beschichtung bestehend aus Aluminiumoxidverbindungen und eine zweite Beschichtung im Wesentlichen bestehend oder bestehend aus Mangan-Verbindungen. Zur Herstellung der Mangan-haltigen Beschichtung werden dabei vorzugsweise Mangan-Verbindung(en) ausgewählt aus den Oxiden, Hydroxiden, Phosphaten, Sulfaten und Fettsäuresalzen des Mangans, wobei es sich bei der Mangan-beschichtung vorzugsweise um Manganoxid handelt, eingesetzt.

Die übrigen Beschichtungen können nach aus dem Stand der Technik bekannten Methoden erzeugt werden. Entsprechende allgemein anwendbare Beschichtungsverfahren sind im Beispielteil exemplarisch angegeben. Vorzugsweise handelt es sich bei den weiteren Beschichtungen um oxidische Beschichtungen des Aluminiums oder Siliciums.

Weiter kann es erfindungsgemäß bevorzugt sein, wenn das partikuläre UV Schutzmittel mit üblichen Methoden, wie beispielsweise in Cosmetics & Toiletries, February 1990, Vol. 105, pp. 53-64 beschrieben, nachbehandelt wird.

Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Weiter kann es erfindungsgemäß bevorzugt sein, wenn das partikuläre UV-Schutzmittel mit üblichen Methoden hydrophobiert wird. Eine Hydrophobierung findet beispielsweise durch Aufbringen einer weiteren organischen Beschichtung oder Schicht statt, wie beispielsweise in WO 2007/065574 beschrieben. Die organische äußere Beschichtung oder Schicht kann beispielsweise aus Silikonölen, Alkylsilanen, olefinischen Säuren, Polyolen oder organophosponischen Säuren oder Mischungen derselben bestehen. Beispielhafte Hydrophobierungskomponenten sind Simethicone, Methicone, Dimethicone/Methicone Copolymer, Trimethoxycarpylsilane, Dimethicone (= Triethoxy-caprylsilane), Diphenyl Capryl Methicone, Alkyl Silane, Silicone, Polymethyl-methacrylate dimethicone, langkettige organische Säuren beispielsweise Stearinsäure, Laurinsäure, Cyclomethicone, Lecithin, Cyclopentasiloxane, Cyclohexasiloxane, Hydroxystearinsäure, hydrogenierte Polydecene, Polyhydroxystearinsäure, Squalen, Octyl Silane (= Trimethoxycaprylsilane), Cetearyl Alkohol, Cetyl_PEG/PPG-10/1 Dimethicone, Dimethicone und PVP/Hexadecene Copolymer, Methicone und PVP/Hexadecene Copolymer, Sodium Hexamethyphosphate, PVP (= polyvinylpyrrolidone), Glyceryl Caprylate/Caprate, Bienenwachs, synthetische Wachse, Microwachse, Isopropyl Titanium Triisostearate/Triethoxycaprylsilane, Isopropyl Titanium Triisostearate/Dimethicone Crosspolymer, Magnesium Myristate, PEG-10 Dimethicone und Methicone, Poly-Acrylate/Methacrylate und Copolymere, Polyglyceryl-3 Diisostearate, Cetyl Dimethicone Copolyol [die Bezeichnungen sind standardisiert, d.h. es sind INCI-Bezeichnungen, die in englischer Sprache festgelegt sind]. Bevorzugt ist eine äußere Beschichtung oder Schicht mit Simethicone, Methicone, Dimethicone, Polysilicon-15, Stearinsäure, Glycerin oder Mischungen derselben. Besonders bevorzugt wäre der Einsatz von Simethicon, Dimethicon oder Stearinsäure, ganz besonders bevorzugt von Simethicon.

Das resultierende partikuläre UV-Schutzmittel zeigt üblicherweise eine Partikelgröße nach der Scherrer-Methode im Bereich von 8 bis 50 nm und insbesondere bevorzugt unterhalb von 25 nm.

Das erfindungsgemäße partikuläre UV-Schutzmittel zeigt dabei vorteilhafte Eigenschaften gegenüber dem Stand der Technik vorzugsweise hinsichtlich:

- UV-Absorption, insbesondere Breitband- bzw. UV-B-Absorption,
- Transparenz im sichtbaren Licht (VIS),
- gute, insbesondere erhöhte Photostabilität,
- verringerte bzw. verhinderte Photoaktivität,
- antioxidante und/oder radikalfangende Eigenschaften
- hydrophile Oberfläche, gute Einarbeitung und Absetzstabilität in wässrigen Phasen;
- leichte Dispergierbarkeit in wässrigen und öligen Phasen und insbesondere eine feine Verteilung in der Dispersion,
- insbesondere wenn es sich um ein erfindungsgemäß bevorzugtes Mittel mit Siliciumdioxid-Beschichtung handelt,

    ◦ in Kombinaton mit Dibenzoylmethan-Derivaten, insbesondere:

        ▪ keine oder reduzierte Auskristallisation von Komplexen der Dibenzoylmethan-Derivate und/oder
        ▪ erhöhte Lagerstabilität der Dibenzoylmethan-Derivate und/oder
        ▪ verbesserte Lichtschutzwirkung, insbesondere nach Lagerung,

    ◦ in Kombination mit Selbstbräunern, insbesondere Dihydoxyaceton, wird keine oder eine gegenüber dem Stand der Technik verminderte Destabilisierung des Selbstbräuners beobachtet,
    ◦ in Kombination mit Benzophenon-Derivaten, insbesondere 2-Hydroxy-4-Methoxy-Benzophenon, wird eine Stabilisierung der Benzophenon-Derivate beobachtet;
    ◦ in Kombination mit Zimtsäure-Derivaten, wie Ethylhexyl-Methoxycinnamate, wird eine Stabilisierung der Zimtsäure - Derivate beobachtet.

Weiter besitzt eine Emulsion enthaltend erfindungsgemäße UV-Schutzmittel typischerweise eine leichte Tönung. Der von der Anwendung Titandioxid-haltiger Sonnencremes bekannte weiße Film auf der Haut, welcher sich teilweise nur schwer bis zur Farblosigkeit verteilen läßt, erhält mit dem erfindungsgemäßen UV-Schutzmittel einen hautähnlichen Farbton. Die Anwendung des Produktes wird somit für den Kunden komfortabler.

Dabei hat es sich insbesondere gezeigt, dass es zur gleichzeitigen Verwirklichung der oben genannten Vorteile vorteilhaft sein kann, wenn das partikuläre Titandioxid mit Cer oder Eisen, vorzugsweise Eisen, dotiert ist.

In einer anderen ebenfals bevorzugten Variante der vorliegenden Erfindung ist das partikuläre Titandioxid jedoch frei von Dotierstoffen.

In einer weiteren Ausführungsform ist ein partikuläres UV-Schutzmittel, wie zuvor beschrieben bevorzugt,

dessen äußere Schicht eine hydrophobierende Schicht ist.

Wie bereits oben erwähnt, werden die partikulären UV-Schutzmittel mit den erfindungsgemäßen Eigenschaften beispielsweise erhalten, wenn ein bestimmtes Herstellverfahren eingehalten wird.

Entsprechend ist ein Verfahren zur Herstellung eines partikulären Titandioxids nach Anspruch 1 mit Lichtschutzeigenschaften, das dadurch gekennzeichnet ist, dass

a) ein partikuläres Titandioxid hydrothermal behandelt wird und
b) anschließend eine Mangen-haltige Beschichtung aufgebracht wird, ein weiterer Gegenstand der vorliegenden Erfindung.

Wie bereits oben ausgeführt kann es bei diesem Verfahren bevorzugt sein, wenn das in Schritt a) eingesetzte partikuläre Titandioxid vorzugsweise mit Eisen dotiert ist.

Die Hydrothermalbehandlung wird dabei vorzugsweise bei Temperaturen im Bereich von 40 bis 360°C, vorzugsweise im Bereich von 80 bis 220°C und insbesondere bevorzugt im Bereich von 140 bis 200°C durchgeführt. In einem bevorzugten Verfahren wird auf eine anschließende Temperung verzichtet.

Durch die Hydrothermalhehandlung kommt es zur Ausbildung stabiler Nano-Kristallite mit gleichmäßiger Größe und Form. Bei niedrigen Temperaturen entstehen "nadelförmige" Kristallite. Mit zunehmender Temperatur verrunden die Kristallite. Es bilden sich ovale Fomen, die bis zu runden Partikeln bei sehr hohen Temperaturen gehen. Zusätzlich kommt es zu einem gleichmäßigen Kristallwachstum, was zu einer Erniedrigung der Reaktivität und Photoaktivität führt.

Vorteile der Hydrothermalbehandlung gegenüber einer üblichen thermischen Behandlung (Temperaturbehandlung eines getrockneten Pulvers) sind:

- Ausbildung gleichmäßiger Kristallitgrößen mit enger Kornverteilung
- Verhinderung von Sintereffekten (Bildung von unerwünschten Aggregaten)

Die Beschichtung in Schritt b) wird vorzugsweise als Sol-Gel-Prozess durchgeführt, wobei insbesondere bevorzugt eine Mangansulfat-Lösung, ggf. zusammen mit weiteren Precursoren zu einer Suspension des Titandioxids gegeben wird.

Dabei wird der Sol-Gel-Prozeß in einer vorteilhaften Variante der vorliegenden Erfindung bei konstant gehaltenem pH-Wert durchgeführt. Der konstant gehaltene pH-Wert kann in einem Bereich von pH 2 bis pH 11 liegen, wobei der pH-Wert vorzugsweise im Bereich von pH = 5 bis pH = 8, insbesonders bevorzugt im Bereich von pH = 6 bis pH = 7 liegt.

Eine weitere vorteilhafte Variante der vorliegenden Erfindung ist die Gesamtzugabe des zur Nachbehandlung notwendigen Wasserglases bei einem pH = 7 bis pH = 11 ohne pH-Konstanthaltung. Anschließend wird der pH-Wert auf einen Wert von pH = 5 bis pH = 8, vorzugsweise auf pH = 6 bis pH = 7 abgesenkt.

Weiter ist es bevorzugt, wenn Schritt b) bei erhöhter Temperatur, vorzugsweise bei einer Temperatur im Bereich von 50°C bis 100°C durchgeführt wird.

Bei allen genannten Varianten des erfindungsgemäßen Verfahrens ist eine Reifezeit nach beendeter Beschichtung vorteilhaft. Die Reifezeit sollte zwischen 1 h und 8 h, bevorzugt 2 h bis 4 h betragen und bei einer Temperatur von 50°C bis 110°C durchgeführt werden. Weiter kann es im Hinblick auf die bei der späteren Verarbeitung erwünschten Agglomeratgrößen von Vorteil sein, wenn das Produkt nachträglich vermahlen wird. Hier können die üblichen bei partikulären Materialen verwendbaren Mahltechniken eingesetzt werden.

In einer bevorzugten Ausführungsform des Verfahrens, wie zuvor beschrieben, wird in einem nachbehandelnden Schritt eine hydrophobierende Schicht aufgebracht.

Weitere bevorzugte Kombinationen von Ausführungsformen sind in den Ansprüchen offenbart.

Ein weiterer Gegenstand der Erfindung sind wässrige oder ölige Dispersionen, enthaltend die erfindungsgemäßen partikulären UV-Schutzmittel. Die Dispersionen können nach herkömmlichen Methoden hergestelllt werden, wie Sie dem Fachmann bekannt sind. Wässrige Disperisonen enthalten vorzugsweise das erfindungsgemäße partikuläre UV-Schutzmittel, wie zuvor beschrieben, Wasser und entsprechende Dispergierhilfsmittel.

Ölige Dispersionen enthalten vorzugsweise das erfindungsgemäße partikuläre UV-Schutzmittel, wie zuvor beschrieben, mindestens ein kosmetisches Öl und entsprechende Dispergierhilfsmittel.

Die Zubereitungen oder Dispersionen können die genannten notwendigen oder optionalen Bestandteile umfassen oder enthalten, daraus im Wesentlichen oder daraus bestehen.

Aufgrund der oben genannten Vorteile ist ein weiterer Gegenstand der vorliegenden Erfindung eine Zubereitung mit Lichtschutzeigenschaften, welche mindestens ein erfindungsgemäßes partikuläres UV-Schutzmittel enthält.

Bei den Zubereitungen handelt es sich in einer Erfindungsvariante vorzugsweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional

weitere geeignete Inhaltsstoffe.

Weitere Zubereitungen können aus der Gruppe Fasern, Textilien, einschließlich deren Beschichtungen, Anstrichstoffe, Beschichtungssysteme, Folien und Verpackungen für den Schutz von Lebensmittel, Pflanzen oder technischen Gütern ausgewählt werden.

Neben den bereits oben genannten Vorteilen kann der Einsatz der erfindungsgemäßen partikulären UV-Schutzmittel in Zubereitungen, die Emulsionen sind, insbesondere auch zur Stabilisierung der Emulsion beitragen. Dadurch kann in der Regel der Einsatz von Emulgatoren verringert werden oder im Einzelfall (Pickering-Emulsion), sogar ganz auf den Einsatz von Emulgatoren verzichtet werden. Erfindungsgemäß bevorzugt sind daher auch emulgatorfreie Emulsionen, welche die erfindungsegemäßen partikulären UV-Schutzmittel enthalten.

In einer Erfindungsvariante enthalten bevorzugte Zubereitungen mit Lichtschutzeigenschaften dabei mindestens ein Dibenzoylmethanderivat. Die im Rahmen der vorliegenden Erfindung verwendeten Dibenzoylmethanderivate sind, wie bereits gezeigt, an sich bereits wohlbekannte Produkte, die insbesondere in den oben genannten Druckschriften FR-A-2 326 405, FR-A-2 440 933 und EP-A-0 114 607 beschrieben sind.

Die verwendbaren Dibenzoylmethanderivate können insbesondere unter den Dibenzoylmethanderivaten der folgenden Formel ausgewählt sein:

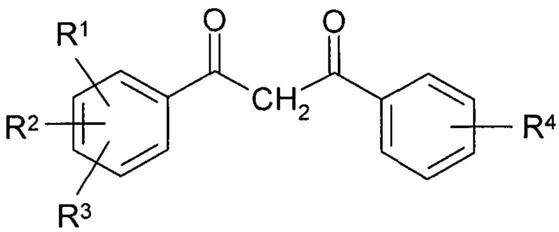

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden. Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,
- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan
  und
- 2,6-Dimethyl-4-tert.-butyl-4'-methoxydibenzoylmethan genannt werden, wobei diese Aufzählung nicht einschränkend ist.

Von den obengenannten Dibenzoylmethanderivaten wird insbesondere das 4,4'-Methoxy-tert.-butyldibenzoylmethan und insbesondere das unter der Handelsbezeichnung Eusolex® 9020 von der Firma Merck im Handel befindliche 4,4'-Methoxy-tert.-butyldibenzoylmethan bevorzugt, wobei dieser Filter der folgenden Strukturformel entspricht:

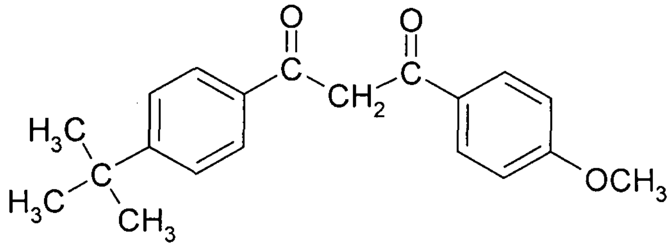

Ein weiteres bevorzugtes Dibenzoylmethanderivat ist das 4-Isopropyldibenzoylmethan.

Weitere bevorzugte Zubereitungen mit Lichtschutzeigenschaften enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenon-derivat(e) können in den erfindungsgemäßen Zusammensetzungen in Mengenanteilen vorliegen, die im Allgemeinen im Bereich von 0,1 Gew.% bis 10 Gew.% liegen und vorzugsweise in Mengenanteilen, die im Bereich von 0,3 Gew.% bis 5 Gew.% liegen, wobei diese Mengenanteile auf das Gesamtgewicht der Zusammensetzung bezogen sind.

Aufgrund der oben genannten Vorteile kann das erfindungsgemäße partikuläre Titandioxid mit Lichtschutzeigenschaften zur Stabilisierung von UV-Filtern, insbesondere Dibenzoylmethan und Derivaten des Dibenzoylmethan bzw. Benzophenon und Derivaten des Benzophenon verwendet werden.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

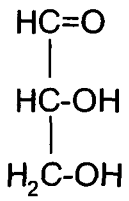

Glycerolaldehyd

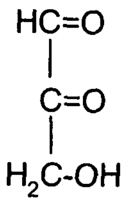

Hydroxymethylglyoxal

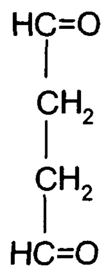

γ-Dialdehyd

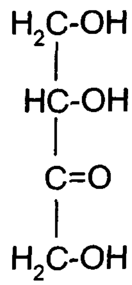

Erythrulose

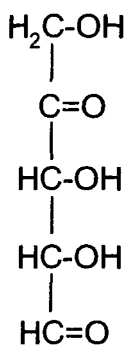

6-Aldo-D-Fructose

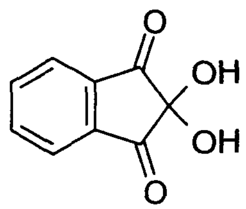

Ninhydrin

Ferner ist das 5-Hydroxy-1,4-naphtochinon (Juglon) zu nennen, das aus den Schalen frischer Walnüsse extrahiert wird

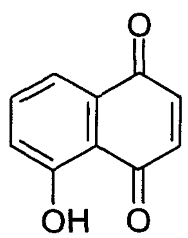

5-Hydroxy-1,4-naphtochinon (Juglon)

sowie das in den Henna-Blättern vorkommende 2-Hydroxy-1,4-naphtochinon (Lawson).

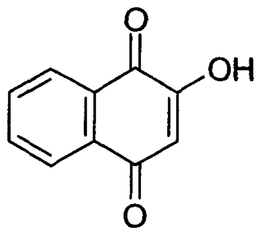

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

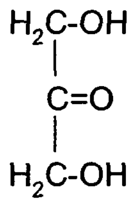

1,3-Dihydroxyaceton (DHA)

Die Verwendung eines erfindungsgemäßen partikulären UV-Schutzmittels zur Stabilisierung von Selbstbräunern, insbesondere Dihydroxyaceton oder Dihydroxyacetonderivaten ist ein weiterer Gegenstand der vorliegenden Erfindung.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäure-anilid | 12490 | Rot |
| Disperse Yellow 16 | 12700 | Gelb |
| 1-(4-Sulfo-1-phenylazo)-4-amino-benzol-5-sulfosäure | 13015 | Gelb |

(fortgesetzt)

| Chemische oder sonstige Bezeichnung | CIN | Farbe |
|---|---|---|
| 2,4-Dihydroxy-azobenzol-4'-sulfosäure | 14270 | Orange |
| -(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |

(fortgesetzt)

| Chemische oder sonstige Bezeichnung | CIN | Farbe |
|---|---|---|
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclo-hexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)-amino-2-methyl-N-ethylIN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylamino-naphthofuchsonimmonium | 44090 | Grün |
| Acid Red 52 | 45100 | Rot |
| 3-(2'-Methylphenylamino)-6-(2'-methyl-4'-sulfophenyl-amino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |

(fortgesetzt)

| Chemische oder sonstige Bezeichnung | CIN | Farbe |
|---|---|---|
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium-und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure,
Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und
sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, ß-Carotin oder Cochenille, insbesondere ß-Carotin.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z. B. Bismuthoxychlorid (BiOCl)

3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung/Schichtdicke** | **Farbe** |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder

Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Erfindungsgemäß können die partikulären UV-Schutzmittel vorzugsweise auch mit einer die hydrophilen oder die hydrophoben Eigenschaften verstärkenden Oberflächenbehandlung versehen sein. Zur hydropoben Modifikation eignet sich beispielsweise eine Silicon- oder Silan-Beschichtung

Die Silicone sind bekanntlich silicium-organische Polymere oder Oligomere mit geradkettiger oder cyclischer, verzweigter oder vernetzter Struktur mit unterschiedlichen Molekülgewichten, die durch Polymerisation und/oder Polykondensation geeignet funktionalisierter Silane erhalten werden und im Wesentlichen aus wiederkehrenden Haupteinheiten gebildet werden, in denen die Siliciumatome über Sauerstoffatome miteinander verknüpft sind (Siloxanbindung), wobei gegebenfalls substituierte Kohlenwasserstoffgruppen über ein Kohlenstoffatom direkt an die Siliciumatome gebunden sind. Die gebräuchlichsten Kohlenwasserstoffgruppen sind die Alkylgruppen und inbesondere Methyl, die Fluoralkylgruppen, die Arylgruppen und insbesondere Phenyl sowie die Alkenylgruppen und insbesondere Vinyl. Weitere Typen von Gruppen, die entweder direkt oder über eine Kohlenwasserstoffgruppe an die Siloxankette gebunden werden können, sind insbesondere Wasserstoff, die Halogene und insbesondere Chlor, Brom oder Fluor, die Thiole, die Alkoxygruppen, die Polyoxyalkylengruppen (oder Polyether) und insbesondere Polyoxyethylen und/oder Polyoxypropylen, Hydroxygruppen oder Hydroxyalkylgruppen, die gegebenenfalls substituierten Aminogruppen, die Amidgruppen, die Acyloxygruppen oder Acyloxyalkylgruppen, die Hydroxyalkylaminogruppen oder Aminoalkylgruppen, quaternäre Ammoniumgruppen, amphotere Gruppen oder Betaingruppen, anionische Gruppen, wie Carboxylate, Thioglykolate, Sulfosuccinate, Thiosulfate, Phosphate und Sulfate, wobei diese Aufzählung selbstverständlich in keiner Weise einschränkend ist (sogenannte 'organomodifizierte' Silicone).

Im Sinne der vorliegenden Erfindung sollen mit dem Ausdruck 'Silicone' auch die zu ihrer Herstellung benötigten Silane und insbesondere die Alkylsilane eingeschlossen und abgedeckt sein.

Die für die vorliegende Erfindung geeigneten Silicone, die zum Umhüllen der partikulären UV-Schutzmittel verwendet werden können, sind vorzugsweise unter den Alkylsilanen, den Polydialkylsiloxanen und den Polyalkylhydrogensiloxanen ausgewählt. Noch bevorzugter sind die Silicone unter Octyltrimethylsilan, den Polydimethylsiloxanen und den Polymethylhydrogenosiloxanen ausgewählt.

Die partikulären UV-Schutzmittel können in den erfindungsgemäßen Zusammensetzungen in Mengenanteilen vorliegen, die im Allgemeinen im Bereich von 0,01 bis 50 Gew.% liegen und vorzugsweise in Mengenanteilen, die im Bereich von 0,5 bis 20 Gew.% liegen, wobei diese Mengenanteile auf das Gesamtgewicht der Zusammensetzung bezogen sind.

Ferner sind auch Kombinationen mit weiteren partikulären UV-Filtern, sowohl als Pulver als auch als Dispersion oder Paste, der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitec® COS.

Weiter kann es erfindungsgemäß bevorzugt sein, wenn die Zubereitungen anorganische UV-Filter enthalten,

die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte weitere partikuläre UV-Filter sind dabei:

- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:

- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Kemira,
- Aluminium und Glycerin; wie z.B. das Produkt UV-Titan der Fa. Kemira,
- Aluminium und Silikonölen, wie z.B. das Produkt UV-Titan M262 der Fa. Kemira,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:

- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
    - o "Zinc Oxide CS-5" der Fa. Toshibi (ZnO nachbehandelt mit polymethylhydrogenosiloxane)
    - o Nanogard Zinc Oxide FN der Fa. Nanophase Technologies
    - o "SPD-Z1" der Fa Shin-Etsu (ZnO nachbehandelt mit einem Silikongepfropften Acrylpolymer, dispergiert in Cyclodimethylsiloxane

o "Escalol Z100" der Fa ISP (Aluminiumoxid nachbehandeltes ZnO dispergiert in einer ethylhexyl methoxycinnamate/PVPhexadecene/methicone copolymer Mischung)

o "Fuji ZNO-SMS-10" der Fa. Fuji Pigment (ZnO nachbehandelt mit Siliciumdioxid und Polymethylsilesquioxan);

o Unbehandeltes Ceroxide Mikropigment z.B. mit der Bezeichnung "Colloidal Cerium Oxide" der Fa Rhone Poulenc

◦ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B . das Produkt UV-Titan M261 der Fa. Kemira in Kombination mit dem erfindungsgemäßen UV-Schutzmittel verwendet werden.

Diese anorganischen UV-Filter werden in der Regel in einer Menge von 0,1 bis 25 Gewichtsprozent, vorzugsweise 2 - 10 %, in kosmetische Zubereitungen eingearbeitet. Insbesondere kann es dabei bevorzugt sein, wenn in Emulsionen in einer Phase ein erfindungsgemäßes nanopartikuläres UV-Schutzmittel und in der anderen Phase ein weiterer anorganischer UV-Filter enthalten ist.

Die erfindungsgemäßen Sonnenschutzmittel können selbstverständlich einen oder mehrere zusätzliche(n) hydrophile(n) oder lipophile(n) Sonnenschutzfilter, die im UV-A-Bereich und/oder UV-B-Bereich und(/oder IR und/oder VIS-Bereich (Absorber) wirksam sind, enthalten. Diese zusätzlichen Filter können insbesondere unter Zimtsäurederivaten, Salicylsäurederivaten, Campherderivaten, Triazinderivaten, β,β-Diphenyl-acrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im folgenden werden die genannten UV-Filter meist nach der INCI-Nomenklatur benannt.

Insbesondere zu nennen sind hier:

- para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Haarmann and Reimer, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Haarmann and Reimer.

β,β-Diphenylacrylate Derivate: Octocrylene, z. B. vetrieben unter dem Namen "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Haarmann and Reimer.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazine Derivate: Ethylhexyltriazone, z. B. vetrieben unter dem Namen "Uvinul T150" von der Fa. BASF,

Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine.

Anthranilin Derivate: Menthyl anthranilate, z. B. vetrieben unter dem Namen "Neo Heliopan MA" von der Fa. Haarmann and Reimer.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindunsgsgemäßen partikulären UV-Schutzmitteln kombiniert werden.

Die zur Kombination mit dem erfindungsgemäßen UV-Schutzmittel geeigneten organischen UV-schützende Substanzen sind bevorzugt aus der folgenden Liste auszuwählen: Ethylhexyl salicylate, Octocrylene, Butylmethoxydibenzoylmethane, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 bis 20 Gewichtsprozent, vorzugsweise 1 - 10 Gew.%, in kosmetische Formulierungen eingearbeitet.

Organische UV-Filter werden insgesamt in der Regel in einer Menge von 0,01 bis 20 Gewichtsprozent, vorzugsweise 0,5-20 %, in kosmetische Formulierungen eingearbeitet.

Bevorzugte Zubereitungen können auch Verbindungen der Formel I enthalten,

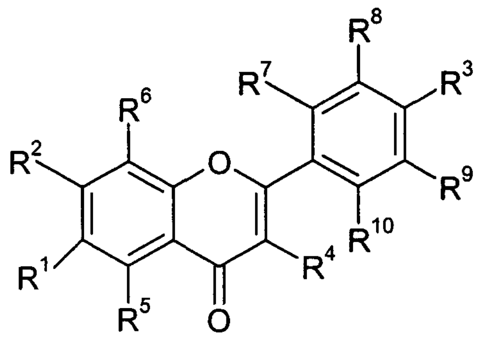

I,

wobei $R^1$ und $R^2$ ausgewählt sind aus

- H
- und $OR^{11}$, wobei $OR^{11}$ unabhängig voneinander steht für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,

- Mono- und/oder Oligoglycosylreste,

mit der Maßgabe, dass mindestens ein Rest aus $R^1$ und $R^2$ steht für $OR^{11}$, und $R^3$ steht für einen Rest $OR^{11}$ und $R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei

  - die Hydroxygruppe an ein primäres oder sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können und
- $R^8$ und $R^9$ gleich oder verschieden sein können, und unabhängig voneinander stehen für
- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können.

Vorteile dieser Zusammensetzungen sind dabei insbesondere die UV-Licht filternde Wirkung und die gute Hautverträglichkeit. Zusätzlich sind die hier beschriebenen Verbindungen der Formel I farblos oder nur schwach gefärbt und führen so, im Gegensatz zu vielen bekannten natürlich vorkommenden Flavonoiden, nicht zu Verfärbungen der Zubereitungen.

Unter den einzusetzenden Flavonoiden der Formel I finden sich dabei Breitband-UV-Filter, andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte Zubereitungen mit Lichtschutzeigenschaften enthalten zumindest eine Verbindung der Formel I, wobei $R^3$ steht für

- OH oder

- geradkettige oder verzweigte $C_1$- bis $C_{20}$-Alkoxygruppen, vorzugsweise Methoxy, Ethoxy oder Ethylhexyloxy oder

- Mono- und/oder Oligoglycosylreste, vorzugsweise Glucosylreste und

- $R^1$ und/oder $R^2$ vorzugsweise stehen für

- OH oder

- geradkettige oder verzweigte $C_1$- bis $C_{20}$-Alkoxygruppen, vorzugsweise Methoxy, Ethoxy oder Ethylhexyloxy oder

- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der einzusetzenden Verbindungen;

- geradkettige oder verzweigte $C_1$- bis $C_{20}$-Alkoxygruppen, insbesondere die langkettigen Alkoxyfunktionen, wie Ethylhexyloxy-Gruppen, erhöhen die Öllöslichkeit der Verbindungen;

d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$- oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^6$ und $R^9$ gleich sind und stehen für H oder geradkettige oder verzweigte $C_1$-bis $C_{20}$-Alkoxygruppen, vorzugsweise Methoxy, Ethoxy oder Ethylhexyloxy.

Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, besonders bevorzugt. Insbesondere ist es dabei bevorzugt, wenn $R^8$ und $R^9$ für H stehen.

Die Verbindungen der Formel I werden typisch in Mengen von 0,01 bis 20 Gew.%, vorzugsweise in Mengen von 0,5 Gew.% bis 10 Gew.% und insbesondere bevorzugt in Mengen von 1 bis 8 Gew.% eingesetzt. Dabei bereitet es dem Fachmann keinerlei Schwierigkeiten die Mengen abhängig von dem beabsichtigten Lichtschutzfaktor der Zubereitung entsprechend auszuwählen.

Durch Kombination von einem partikulären oder mehrerer partikulärer UV-Schutzmittel mit weiteren UV-Filtern kann die Schutzwirkung gegen schädliche Einwirkungen der UV-Strahlung optimiert werden. Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.

Alle genannten UV-Filter einschließlich der Verbindungen der Formel I können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung, vermieden werden, da die Wechselwirkung unterbunden wird.

Daher kann es bevorzugt sein, wenn ein oder mehrere der Verbindungen gemäß Formel I bzw. der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxidhydroxid) aufgebaut sind. Die Herstellung entsprechender Kapseln ist dem Fachmann beispielsweise aus den zitierten Patentanmeldungen bekannt.

Dabei sind die Kapseln in Zubereitungen vorzugsweise in solchen Mengen enthalten, die gewährleisten, dass

die verkapselten UV-Filter in den oben angegebenen Mengen in der Zubereitung vorliegen.

Weisen die Zubereitungen Verbindungen entsprechend Formel I mit freien Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Allgemein wirken die erfindungsgemäßen UV-Schutzmittel als Antioxidans, insbesondere über ihre Wirkung als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalanionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass erfindungsgemäße UV-Schutzmittel auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.

Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse.

Die antioxidante Wirkung der erfindungsgemäßen UV-Schutzmittel läßt sich mit verschiedenen Tests untersuchen. Nachweismethoden für die erfindungsgemäßen Eigenschaften sind im Beispielteil angegeben, und lassen sich nicht nur für die konkret dort angegebenen Beispiele sondern ganz generell anwenden.

Insbesondere bevorzugt ist es, wenn der dekadische Extinktionskoeffizient bei 300nm eines Lackes enthaltend das UV Schutzmittel größer als 30, vorzugsweise größer als 35 und insbesondere bevorzugt größer als 40 ist. Weiter kann es bevorzugt sein, wenn der dekadische Extinktionskoeffizient bei 560 nm eines Lackes enthaltend das UV Schutzmittel kleiner als 1 ist.

Erfindungsgemäße Zusammensetzungen können sich auch zur Behandlung von Hautkrankheiten eignen, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkrankungen, wie Arteriosklerose oder Bluthochdruck, sowie des Insulinunabhängigen Diabetes, zur Behandlung von Hautproblemen, die durch UV-Strahlung hervorgerufen werden.

Die schützende Wirkung gegen oxidativen Stress bzw. gegen die Einwirkung von Radikalen kann weiter verbessert werden, wenn die Zubereitungen ein oder mehrere Antioxidantien enthalten.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie vorzugsweise ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall-) Chelatoren, (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-$\alpha$-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-$\alpha$-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-$\alpha$-Tocopherol, Tocopherol-E-Acetat, Nicotinsäure, Pantothensäure und Biotin.

Neben den hier beschriebenen Verbindungen können die erfindungsgemäßen Zubereitungen in einer besonders bevorzugten Variante auch mindestens eine Verbindung entsprechend der Formel II als Antioxidans enthalten,

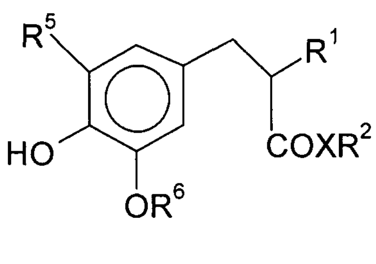

II,

wobei

$R^1$ ausgewählt ist aus $-C(O)CH_3$, $-CO_2R^3$, $-C(O)NH_2$ and $-C(O)N(R^4)_2$;

X is O or NH;

$R^2$ steht für einen linearen oder verzweigten $C_{1-30}$-Alkylrest;

$R^3$ steht für einen linearen oder verzweigten $C_{1-20}$-Alkylrest,

alle $R^4$ unabhängig voneinander stehen für H oder lineare oder verzweigte $C_{1-8}$-Alkylreste

$R^5$ steht für H, einen linearen oder verzweigten $C_{1-8}$-Alkylrest oder einen linearen oder verzweigten -O-$C_{1-8}$-Alkylrest und

$R^6$ steht für einen $C_{1-8}$-Alkylrest.

Bei dem Antioxidans gemäß Formel II handelt es sich insbesondere bevorzugt um 4-Hydroxy-3,5-dimethoxy-benzyl-malonsäure-di-2-ethyl-hexylester (RonaCare® AP). Entsprechende Antioxidantien, ihre Herstellung und Verwendung sind in der Internationalen Patentanmeldung WO 2006/111233 beschrieben.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle *(E. A. Galinski et al., Eur. J. Biochem., 149 (1985) Seite 135-139).* Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel III eingesetzt,

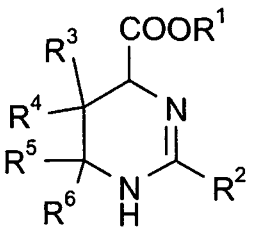

III,

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist

bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.% des Aryloxims, wobei es insbesondere bevorzugt ist, wenn die Zubereitung 0,05 bis 5 Gew-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Neben den hier beschriebenen Verbindungen können die erfindungsgemäßen Zubereitungen auch mindestens einen Photostabilisator, vorzugsweise entsprechend der Formel IV

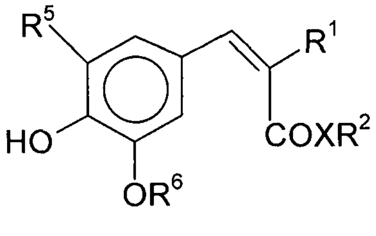

IV,

wobei

$R^1$ ausgewählt ist aus $-C(O)CH_3$, $-CO_2R^3$, $-C(O)NH_2$ and $-C(O)N(R^4)_2$;

X is O or NH;

$R^2$ steht für einen linearen oder verzweigten $C_{1-30}$-Alkylrest;

$R^3$ steht für einen linearen oder verzweigten $C_{1-20}$-Alkylrest,

alle $R^4$ unabhängig voneinander stehen für H oder lineare oder verzweigte $C_{1-8}$-Alkylreste

$R^5$ steht für H, einen linearen oder verzweigten $C_{1-8}$-Alkylrest oder einen linearen oder verzweigten $-O-C_{1-8}$-Alkylrest und

$R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben.

Die erfindungsgemäßen Zusammensetzungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Weiterer Gegenstand der vorliegenden Erfindung ist ein Verfahren zur Herstellung einer Zubereitung, welches dadurch gekennzeichnet ist, dass mindestens ein partikuläres UV-Schutzmittel mit einem kosmetisch oder dermatologisch geeigneten Träger vermischt wird.

Diese Zusammensetzungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zusammensetzungen in Form einer O/W-Emulsion vor.

Die erfindungsgemäßen kosmetischen Zusammensetzungen können als Zusammensetzungen zum Schutz der menschlichen Epidermis und/oder der Haare gegen UV-Strahlung, als Sonnenschutzmittel, in der Tagespflege oder als Schminkprodukte verwendet werden.

Es soll darauf hingewiesen werden, dass in den erfindungsgemäßen Formulierungen zum Sonnenschutz, die einen Träger vom Typ einer Öl-in-Wasser-Emulsion aufweisen, die wässerige Phase (die insbesondere die hydrophilen Filter enthält) im Allgemeinen 50 bis 95 Gew.% und vorzugsweise 70 bis 90 Gew.%, bezogen auf die gesamte Formulierung, die Öl-phase (die insbesondere die lipophilen Filter enthält) 5 bis 50 Gew.% und vorzugsweise 10 bis 30 Gew.%, bezogen auf die gesamte Formulierung und der (Co)emulgator oder die (Co)emulgatoren 0,5 bis 20 Gew.% und vorzugsweise 2 bis 10 Gew.%, bezogen auf die gesamte Formulierung, ausmachen.

Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten

Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Kosmetische Formulierungen welche auch Bestandteil dieser Erfindung sind können ganz generell gängige kosmetische Inhaltsstoffe und Zusätze besonders der folgenden Substanzen bzw. Substanzklassen beinhalten:

Fetthaltige Substanzen, kosmetische Öle (synthetisch und/oder natürlich), organische Lösungsmittel, sowohl nichtionisch als auch ionisch (u.a sogenannte ionische Flüssigkeiten), hydrophile, lipophile oder amphiphile Verdicker, Enthärter, Feuchthaltemittel, Trübungsmittel, Stabilisatoren, Silikonöle und Silikonölderivate, Antschäumungsmittel, Parfum, Konservierungsmittel, anionische, kationisch, nichtionische, zwitterionische Tenside, kosmetische Wirkstoffe, Füllstoffe, Polymere, Treibgase, Säuren und /oder Laugen, und jede belibige Substanz die in der Kosmetik im Allgemeinen verwendet wird.

Fetthaltige Substanzen können Öle oder Wachse oder Mischungen daraus sein. Mit dem Begriff Öl sind Substanzen & Verbindungen gemeint welche bei Raumtemperatur flüssig sind. Mit dem Bergiff Wachse, sind Substanzen & Verbindungen gemeint welche eine feste oder halbfeste Konsistenz haben und deren Schmelzpunkt >35°C liegt.

Der Begriff Öle beinhaltet Mineralöle (Paraffinöle), Pflanzenöle (wie z.B. Jojoba Öl), synthetische Öle wie z.B. Perhydrosqualene, Fettalkohole, Fettsäuren oder Fettsäureester wie z.B. das C12-C15 alkyl benzoate im Handel Unter dem Markennamen "Witconol TN" von der Fa. Witco, octyl palmitate, isopropyl lanolate und Triglyceride, eingeschlossen dem capric/caprylic acid triglycerides, Silikonöle (cyclomethicone and polydimethylsiloxanes, oder PDMS) oder Fluoro öle, and Polyalkylene.

Wachsbestandteile einer kosmetischen Formulierung können z.B. Paraffin Wachs, Carnauba Wachs, Bienenwachs, oder Hydrogeniertes Ricinusöl sein.

Zu den möglichen organischen Lösungsmitteln gehören unter anderem niedere Alkohole und Polyole. Polyole können z.B. aus den folgenden Substanzen/Substanzklassen ausgewählt sein: Glycerin, Glykolether, Ethylenglykol, Propylenglykol, Bytulenglykol, Dipropylenglykol, Diethylenglykol.

Hydrophile Verdicker welche in Kombination mit dem erfindungsgemäßen UV-Schutzmittel kombiniert werden können, sind beispielhaft aus der Gruppe der folgenden ausgewählt: Carboxyvinypolymere wie z.B. Carbopole (carbomere) der Fa. Noveon sowie Pemulen-Produkte (acrylate/C10-C30-alkylacrylate copolymer); Polyacrylamide, wie z.B. das quervenetzte copolymer mit dem Handelsnamen Sepigel 305 (CTFA name: polyacrylamide/C13-14 isoparaffin/Laureth 7) oder Simulgel 600 (CTFA name: acrylamide/sodium acryloyldimethyltaurate copolymer/ isohexadecane/polysorbate 80) der Fa. SEPPIC; 2-acrylamido-2-methyl-propansulfonsäurepolymer und Copolymer, welches optional quervernetzt oder neutralisiert sein kann wie z.B. poly(2-acrylamido-2-methylpropan-sulfonsäure) vertrieben unter dem Handelsnamen "Hostacerin AMPS" (CTFA name: ammonium polyacryidimethyltauramide); Cellulose-basierte Derivate wie z.B. die Hydroxyethylcellulose; Polysaccharide und im besonderen Xanthan Gummi; und Mischungen daraus.

Im folgenden genannte lipophile Verdicker können auch in Kombination mit der erfindunggemäßen Substanz einsegetzt werden z.B: Modifizierte Tonerde wie z.B Hectorite deren Derivate.

Folgende kosmetischen Wirkstoffe können alleine oder in Kombination eingesetzt werden:

Schadstoffabweisende und/oder Radikalfänger;

Hautaufhellende und/oder Hautbräunende Mittel;

Anti-glycation Wirkstoff;

NO-Synthese Inhibitoren;

Wirkstoffe zur Stimulation der Synthese von dermalen und epidermalen Makromolekülen und/oder zum Schutze des Abbaus dieser Verbindungen Wirkstoffe zur Stimulation der Fibroplastenproliferation;

Wirkstoffe zur Stimulation der Keratinocytenproliferation;

Muskelentspannende Wirkstoffe;

Spannungsaufbauende und reduzierende Wirkstoffe;

Hautschuppenabtragende Wirkstoffe;

Feuchthaltemittel;

Antientzündliche Wirkstoffe;

Wirkstoffe welche positive auf den Energiemetabolismus der Zelle wirken;

Instektenabwehrende Mittel;

Substanz P oder CGRP Antagonisten.

Emulgatoren welche besonders bevorzugt zum Einsatz für die Herstellung von WO/ Emulsionen und Cremes kommen sind unter anderem folgende:

Sorbitan glycerin und/oder Zuckeralkylester oder Ether; Silicontenside, wie z.B. Mischung aus Dimethicone copolyol, vetrieben unter dem Handelsnamen "DC 5225 C" von der Fa. Dow Corning, und Alkyldimethicone Copolyole wie z.B. das Laurylmethicone copolyol vetrieben unter dem Handelsnamen "Dow Corning 5200 Formulation Aid" von der Fa. Dow Corning; Cetyldimethicone copolyol, wie z.B. das Handelsprodukt Abil EM 90R von der Fa. Goldschmidt, Mischung aus Cetyidimethicone copolyol, bestehend aus Polyglyceryl isostearate (4 mol) und Hexyl laurate, vetrieben unter dem Handelsnamen Abil WE 09 von der Fa. Goldschmidt. Einer oder mehrere Coemulgatoren können außerdem in Kombination verwendet Polyolalkylester z.B. Glycerin und/oder Sorbitanester, z.B: Polyglyceryl isostearatewelches im Handel unter dem Namen Isolan Gl 34 von der Fa. Goldschmidt vetrieben wird; Sorbitan isostearate, wie z.B. vetrieben unter dem Namen Arlacel 987 von der Fa. Uniqema (Croda); Sorbitan glyceryl isostearate, vetrieben unter dem Handelsnamen Arlacel 986 von der Fa. UUUniqema (Croda) und Mischungen daraus.

Beispielhaft als Emulgatoren für O/W Emulsionen eignen sich nichtionische emulgatoren wie z.B ethoxylierte (im speziellen polyethoxylierte) Fettsäureester des Glycerins, Ethoxylierte Sorbitanfettsäureester; Ethylen und/oder Propylenoxylierte Fettsäureester, von Zucker, wie z.B. Sucrose Stearate; Fettalkoholether des Zuckers, wie z.B. Polyalkylglucoside (APG) wie z.B. Decylglucosilde und Laurylglucoside wie z.B. unter dem Handelsnamen Plantaren von der Fa. Cognis erhältlich. Enthalten sein können auch Cetostearyl glucoside pur oder als Mischung wie z.B. in dem Handelsprodukt Montanov 68 von der Fa. Seppic; TegoCare CG 90 (Fa. Goldschmidt); Emulgade KE3302 (Cognis/Henkel). Mögliche O/W Emulgatoren bilden auch Vebindungen der Arachidyl glucoside, wie z.B. als Mischung mit Aarachidyl alcohol, Behenyl alcohol und Arachidyl glucoside, vetrieben unter dem Handelsnamen Montanov 202 von der Fa. SEPIC.

Zusammensetzungen enthaltend das erfindungsgemäßes UV-Schutzmittel haben ein breites Einsatzgebiet speziell in der pflegenden und dekorativen Kosmetik. Diese sind geeignet zum Schutze der Haut, der Lippen, des Haares, der Kopfhaut, der Hände, der Nägel, Augenbrauen, der Augenlider, im speziellen zum Schutze der beschrieben Areale vor photo-und oder oxidativ induziertem Stress.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl

eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

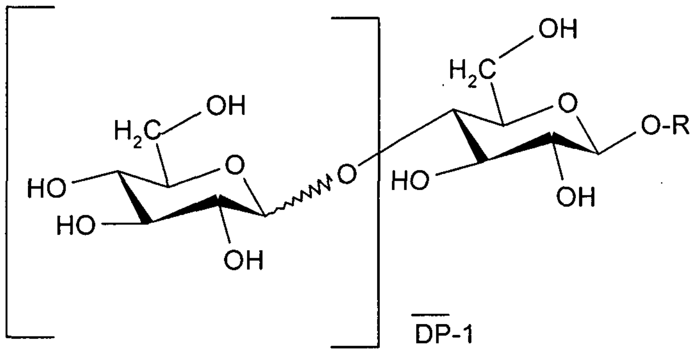

auszeichnen, wobei R einen verzweigten oder unverzweigten Alkylrest mit 4 bis 24 Kohlenstoffatomen darstellt und wobei DP einen mittleren Glucosylierungsgrad von bis zu 2 bedeutet. Der Wert DP repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.%.

Besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die

Strukturformel

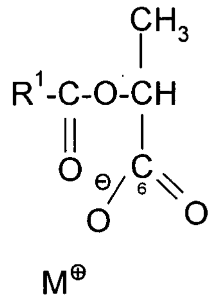

auszeichnen, wobei $R^1$ einen verzweigten oder unverzweigten Alkylrest mit 1 bis 30 Kohlenstoffatomen bedeutet und $M^+$ aus der Gruppe der Alkali-ionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht. Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

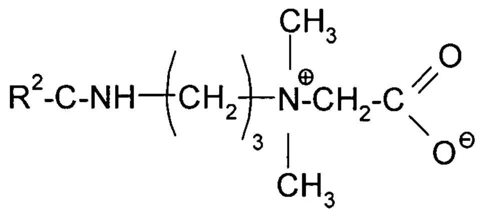

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.% bevorzugt 0,05-10 Gew.%, besonders bevorzugt 0,1-5 Gew.%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Als besonders bevorzugter Emulgator für O/W-Emulsionen hat sich das Handelsprodukt Ceralution C (Geminitensid) der Firma Sasol erwiesen.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylen-glycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)iso-cetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol-(19)isocetylether (Isoceteth-19), Polyethylenglycol(20)isocetylether (Isoceteth-20), Polyethylenglycol(12)oleylether (Oleth-12), Polyethylen-glycol(13)oleylether (Oleth-13), Polyethylenglycol(14)oleylether (Oleth-14), Polyethylenglycol(15)oleylether (Oleth-15), Polyethylenglycol(12)lauryl-ether (Laureth-12), Polyethylenglycol(12)isolaurylether (Isolaureth-12), Polyethylenglycol(13)cetylstearylether (Ceteareth-13), Polyethylen-glycol(14)cetylstearylether (Ceteareth-14), Polyethylenglycol(15)cetyl-stearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethytenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen. Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen UV-induzierte Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Nachfolgend sein hier noch einzelne Emulgatoren beispielhaft für die beschriebenen chemischen Substanzklassen genannt, die in Kombination mit dem erfindungsgemäßen Produkt zur Herstellung von Zubereitungen zum Schutz der Haut und der Haare eingesetzt werden können.

**Die nachfolgenden Produkte sind Handelsmarken der Fa. Degussa Goldschmidt:**

| | |
|---|---|
| Abil Care 85 | Dimethicone Copolyol(and)Caprylic/Capric Triglycerides |
| Abil EM 90 | Cetyl PEG/PPG-10/1 Dimethicone |
| Abil EM 97 | Bis-PEG/PPG-14/14 Dimethicone (and) Cyclopentasiloxane |
| Abil WE 09 | Polyglyceryl-4 Isostearate (and) Cetyl Dimethicone Copolyol (and) Hexyl Laurate |
| Tego Care 150 | GlycerylStearate(and)Steareth-25(and)Ceteth-20(and)StearylAlcohol |
| Tego Care 215 | Ceteareth-15 (and) Glyceryl Stearate |
| Tego Care 450 | Polyglyceryl-3 Methylglucose Distearate |
| Tego Care CG 90 | Cetearyl Glucoside |
| Tego Care PS | Methyl Glucose Sesquistearate |
| TEGO Care 165 | Glyceryl Stearate (and) PEG-100 Stearate |
| ISOLAN GPS | Polyglyceryl-4 Diisostearate and Polyhydroxystearate Sebacate |
| TEGO Care CE 40 | Cetearyl Alcohol; Palmitamidopropyltrimonium Chloride |
| TEGO SIS 40 | PEG-40 Sorbitanperisostearate |

**Die nachfolgenden Produkte sind Handelsmarken der Fa. Cognis Deutschland:**

| | |
|---|---|
| Emulgade F | Cetearyl Alcohol (and) PEG-40 Castor Oil (and) Sodium Sulfate |
| Emulgade 1000Ni | Cetearyl Alcohol(and)Ceteareth-20 |
| Emulgade CM | Cetearyl Isononanoate (and) Ceteareth-20 (and) |

(fortgesetzt)

| | |
|---|---|
| | Cetearyl Alcohol (and) Glyceryl Stearate (and) Glycerin (and) Ceteareth-12 (and) Cetyl Palmitate |
| Eumulgin VL 75 | Lauryl Glucoside(and)Polyglyceryl-2 Dipolyhydroxystearate(and)Glycerin |
| Emulgade sucro | Sucrose Polystearate (and) Hydrogenated Polyisobutene |
| Eumulgin SG | Sodium Stearoyl Glutamate |
| Dehymuls HRE-7 | PEG-7 Hydrogenated Castor Oil |
| Dehymuls LE | PEG-30 Dipolyhydroxystearate |
| Dehymuls PGPH | Polyglyceryl-2 Dipolyhydroxystearate |

**Die nachfolgenden Produkte sind Handelsmarken der Unigema, Belgien**

| | |
|---|---|
| ARLATONE 2121 | Sorbitan Stearate (and) Sucrose Cocoate |
| ARLATONE LC | Sorbitan Stearate (and) Sorbityl Laurate ARLATONE V-100 Steareth-100 (and) Steareth-2 (and) Glyceryl Stearate Citrate (and) Sucrose (and) Mannan (and) Xanthan Gum |
| ARLATONE V-175 | Sucrose Palmitate (and) Glyceryl Stearate (and) Glyceryl Stearate Citrate (and) Sucrose (and) Mannan (and) Xanthan Gum |
| ARLACEL 1689V | Sorbitane Oleate (and) Polyglyceryl-3 Polyricinoleate |
| ARLACEL 1690 | Sorbitan Isostearate (and) Polyglyceryl-3 Polyricinoleate |
| ARLACEL 186 | Glyceryl Oleate (and) Propylene Glycol |
| ARLACEL 481V | Sorbitan Oleate (and) Hydrogenated Castor Oil (and) Beeswax (and) Stearic Acid |
| ARLACEL 582 | Sorbitan Isostearate (and) PEG-2 Hydrogenated Castor Oil (and) Ozokerite (and) Hydrogenated Castor Oil Solid |
| ARLACEL 83V | Sorbitan Sesquioleate |
| ARLACEL 986 | Sorbitan Isostearate (and) Hydrogenated Castor Oil (and) Beeswax (and) Stearic Acid |
| ARLACEL 987 | Sorbitan Isostearate |
| ARLACEL 989 | PEG-7 Hydrogenated Castor Oil |
| ARLACEL P135 | PEG-30 Dipolyhydroxystearate |
| PRISORINE 3700 | Polyglyceryl-3 Diisostearate |
| PRISORINE 3791 | Polyglyceryl-2 Isostearate |
| SPAN 20 | Sorbitan Laurate |
| SPAN 80V | Sorbitan Oleate |
| SPAN 85V | Pharma Sorbitan Trioleate Liquid |

**Die nachfolgenden Produkte sind Handelsmarken der Fa. Tri-K Ind.:**

| | |
|---|---|
| Biobase EP | Glyceryl Stearate, Cetearyl Alcohol, Sodium Stearoyl Lactylate, Lecitin |
| Biobase RS | Glyceryl Stearate, Cetearyl Alcohol, Sodium Stearoyl Lactylate, Tocopherol |

**Die nachfolgenden Produkte sind Handelsmarken der Fa Varma FarmaCosmetica Srl**

| | |
|---|---|
| Emulvama AGC | Glyceryl Stearate, Cetearyl Alcohol, Stearic Acid, Sodium Cocoyl Glutamate |
| Emulvama AGC | Glyceryl Stearate, Cetearyl Alcohol, Stearic Acid, Sodium Cocoyl Glutamate |
| Emulvama AGW | Sodium Cocoyl Glutamate, Sodium Cocoyl Hydrolyzed Wheat Protein, Disodium Capryloyl Glutamate, Potassium Cocoyl PCA |

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Im folgenden wird die Erfindung anhand von Beispielen näher erläutert.

## Beispiele

### Beispiel 1a Herstellung von nano-$TiO_2$

710 mL Natriumtitanat (Gehalt 100 g $TiO_2$/L), erhalten durch Umsetzung von Metatitansäure mit Natronlauge, wird mit 100mL Wasser verdünnt und durch Zugabe von Salzsäure unter Bildung von Titandioxid (Rutil) bei pH 2,5 zersetzt. Dieses durch die Zersetzung erhaltene partikuläre Titandioxid wird unter Zugabe von 115 mL 30%iger Salzsäure peptisiert und durch weitere Wasserzugabe auf 1000 mL-Gesamtvolumen ergänzt. Die Peptisation erfolgt in einem geschlossenen Glaskolben bei 105°C über einem Zeitraum von 2 h. Das Produkt zeigt nadelförmige Kristallite.

### Vergleichsbeispiel 1b Herstellung von nano-$TiO_2$

Das Versuchsprodukt aus Beispiel 1a wird auf eine Filtratleitfähigkeit kleiner 100 μS/cm gewaschen und getrocknet. Vergleichsbeispiel

### 2a Beschichtung von Nano-$TiO_2$ mit $Al_2O_3$

1L des Versuchsproduktes aus Beispiel 1 a wird nach Beendigung der Peptisierung mit Natronlauge auf pH=7 eingestellt und auf 80°C erhitzt. Anschließend werden 40mL Natriumaluminat - Lösung (Gehalt entspricht 300g $Al_2O_3$/L) bei konstantem pH-Wert (pH=7; Regelung durch Zugabe von $H_2SO_4$) zugegeben. Nach erfolgter Zugabe wird bei pH=7 und 80°C 2 Stunden gerührt.

### Vergleichsbeispiel 2b Beschichtung von Nano-$TiO_2$ mit $Al_2O_3$

Das Versuchsprodukt aus Beispiel 2a wird auf eine Filtratleitfähigkeit kleiner 100μS/cm gewaschen und getrocknet.

### Beispiel 3a Beschichtung von Nano-$TiO_2$ mit $Al_2O_3$ und anschließend $MnO_2$

1 L des Versuchsproduktes aus Beispiel 2a werden auf 80°C erhitzt. Anschließend werden 100mL einer $MnSO_4$-Lösung (2g Mn/L) bei konstantem pH-Wert (pH=7; Regelung durch Zugabe von NaOH) zugegeben. Die Suspension wird für weitere 30min gerührt. Das Produkt wird anschließend auf eine Filtratleitfähigkeit kleiner 100 μS/cm gewaschen und getrocknet.

**Beispiel 3b Beschichtung von Nano-$TiO_2$ mit $MnO_2$ und anschließend $Al_2O_3$**

1 L des Versuchsproduktes aus Beispiel 1 a wird nach Beendigung der Peptisierung auf 80°C erhitzt. Anschließend werden 100mL einer $MnSO_4$-Lösung (2g Mn/L) zugegeben. Nach einer Rührzeit von 30min wird die Suspension mit Natronlauge auf pH=7 eingestellt. Anschließend werden 40mL Natriumaluminat - Lösung (entsprechend 300g $Al_2O_3$/L) bei konstantem pH-Wert (pH=7; Regelung durch Zugabe von $H_2SO_4$) zugegeben. Nach erfolgter Zugabe wird bei pH=7 und 80°C 2 Stunden gerührt. Anschließend wird das Produkt auf eine Filtratleitfähigkeit kleiner 100μS/cm gewaschen und getrocknet.

**Beispiel 3c Beschichtung von Nano-$TiO_2$ mit einer $Al_2O_3$ und $MnO_2$-Mischung**

1 L des Versuchsproduktes aus Beispiel 1 a werden auf 80°C erhitzt und mit Natronlauge auf pH=7 neutralisiert. Anschließend werden gleichzeitig 40mL Natriumaluminat-Lösung (entsprechend 300g $Al_2O_3$/L) und 100mL einer $MnSO_4$-Lösung (2g Mn/L) bei konstantem pH-Wert (pH=7; Regelung durch Zugabe von $H_2SO_4$) zugegeben. Nach erfolgter Zugabe wird bei pH=7 und 80°C 2 Stunden gerührt. Anschließend wird das Produkt auf eine Filtratleitfähigkeit kleiner 100μS/cm gewaschen und getrocknet.

**Beispiel 3d Beschichtung von Nano-$TiO_2$ mit $Al_2O_3$ und anschließend Manganphosphat**

1 L des Versuchsproduktes aus Beispiel 2a werden auf 80°C erhitzt. Anschließend werden gleichzeitig 100mL einer $MnSO_4$-Lösung (2g Mn/L) und 100mL einer $Na_3PO_4$-Lösung (4g $Na_3PO_4$/L) bei konstantem pH-Wert (pH=7; Regelung durch Zugabe von NaOH/$H_2SO_4$) zugegeben. Die Suspension wird für weitere 30min gerührt. Das Produkt wird anschließend auf eine Filtratleitfähigkeit kleiner 100 μS/cm gewaschen und getrocknet.

**Beispiel 4a Beschichtung von Nano-$TiO_2$ mit $Al_2O_3$ und anschließend $MnO_2$**

1 L des Versuchsproduktes aus Beispiel 2a werden auf 80°C erhitzt. Anschließend werden 100mL einer $MnSO_4$-Lösung (5g Mn/L) bei konstantem pH-Wert (pH=7; Regelung durch Zugabe von NaOH) zugegeben. Die Suspension wird für weitere 30min gerührt. Das Produkt wird anschließend auf eine Filtratleitfähigkeit kleiner 100 μS/cm gewaschen und getrocknet.

**Beispiel 4b Beschichtung von Nano-$TiO_2$ mit $MnO_2$ und anschließend $Al_2O_3$**

1 L des Versuchsproduktes aus Beispiel 1 a wird nach Beendigung der Peptisierung auf 80°C erhitzt. Anschließend werden 100mL einer $MnSO_4$-Lösung (5g Mn/L) zugegeben. Nach einer Rührzeit von 30min wird die Suspension mit Natronlauge auf pH=7 eingestellt. Anschließend werden 40mL Natriumaluminat-Lösung (entsprechend 300g $Al_2O_3$/L) bei konstantem pH-Wert (pH=7; Regelung durch Zugabe von $H_2SO_4$) zugegeben. Nach erfolgter Zugabe wird bei pH=7 und 80°C 2 Stunden gerührt. Anschließend wird das Produkt auf eine Filtratleitfähigkeit kleiner 100μS/cm gewaschen und getrocknet.

**Beispiel 4c Beschichtung von Nano-$TiO_2$ mit einer $Al_2O_3$ und $MnO_2$-Mischung**

1 L des Versuchsproduktes aus Beispiel 1 a werden auf 80°C erhitzt und mit Natronlauge auf pH=7 neutralisiert. Anschließend werden gleichzeitig 40mL Natriumaluminat-Lösung (entsprechend 300g $Al_2O_3$/L) und 100mL einer $MnSO_4$-Lösung (5g Mn/L) bei konstantem pH-Wert (pH=7; Regelung durch Zugabe von $H_2SO_4$) zugegeben. Nach erfolgter Zugabe wird bei pH=7 und 80°C 2 Stunden gerührt. Anschließend wird das Produkt auf eine Filtratleitfähigkeit kleiner 100μS/cm gewaschen und getrocknet.

**Vergleichsbeispiel 5a Temperung**

100g des Versuchsproduktes aus Beispiel 2b werden 2h bei 400°C getempert.

**Beispiel 5b Temperung**

100g des Versuchsproduktes aus Beispiel 3a werden 2h bei 400°C getempert.

**Beispiel 5c Temperung**

100g des Versuchsproduktes aus Beispiel 4a werden 2h bei 400°C getempert.

**Beispiel 5d Temperung**

100g des Versuchsproduktes aus Beispiel 3d werden 2h bei 400°C getempert.

**Beispiel 6a: Beschichtung des nano-$TiO_2$ mit $SiO_2$ anschließend $MnO_2$**

1 L der wässrigen, salzsauren Suspension des $TiO_2$ aus Beispiel 1 b wird mit NaOH auf einen pH-Wert von 6,5 gebracht und auf 80°C erhitzt. Anschließend werden zu der Suspension 52 mL Wasserglaslösung (entsprechend 384 g $SiO_2$ / L) bei konstantem pH-Wert (pH = 6,5 ± 0,5 ; Regelung durch Zugabe von $H_2SO_4$) zugegeben. Nach erfolgter Zugabe wird bei pH = 6,8 und 80°C 2 Stunden gerührt. Anschließend werden bei 80°C 100mL einer $MnSO_4$-Lösung (5g Mn/L) bei konstantem pH-Wert (pH=7; Regelung durch Zugabe von NaOH) zugegeben. Die Suspension wird für weitere 30min gerührt. Das Produkt wird anschließend auf eine Leitfähigkeit kleiner 100 μS/cm gewaschen und getrocknet.

**Beispiel 6b: Beschichtung des nano-$TiO_2$ mit $SiO_2$ anschließend $MnO_2$**

1 L der wässrigen, salzsauren Suspension des $TiO_2$ aus Beispiel 1 b wird mit NaOH auf einen pH-Wert von 9,0 gebracht und auf 80°C erhitzt. Anschließend werden zu der Suspension 52 mL Wasserglaslösung (entsprechend 384 g $SiO_2$ / L) bei konstantem pH-Wert (pH = 9,0 ± 0,5, Regelung durch Zugabe von $H_2SO_4$) zugegeben. Nach erfolgter Zugabe wird bei pH = 6,8 und 80°C 2 Stunden gerührt. Anschließend werden bei 80°C 100mL einer MnSO4-Lösung (5g Mn/L) bei konstantem pH-Wert (pH=7; Regelung durch Zugabe von NaOH) zugegeben. Die Suspension wird für weitere 30min gerührt. Das Produkt wird anschließend auf eine Leitfähigkeit kleiner 100 μS/cm gewaschen und getrocknet.

**Beispiel 6c: Beschichtung des nano-$TiO_2$ mit $SiO_2$ anschließend $MnO_2$**

1 L der wässrigen, salzsauren Suspension des $TiO_2$ aus Beispiel 1 b wird mit NaOH auf einen pH-Wert von 2,0 gebracht und auf 80°C erhitzt. Anschließend werden zu der Suspension 52 mL Wasserglaslösung (entsprechend 384 g $SiO_2$ / L) bei konstantem pH-Wert (pH = 2,0 ± 0,5, Regelung durch Zugabe von $H_2SO_4$) zugegeben. Nach erfolgter Zugabe wird bei pH = 6,8 und 80°C 2 Stunden gerührt. Anschließend werden bei 80°C 100mL einer $MnSO_4$-Lösung (5g Mn/L) bei konstantem pH-Wert (pH=7; Regelung durch Zugabe von NaOH) zugegeben. Die Suspension wird für weitere 30min gerührt. Das Produkt wird anschließend auf eine Leitfähigkeit kleiner 100 μS/cm gewaschen und getrocknet.

**Beispiel 6d: Beschichtung des nano-$TiO_2$ mit $SiO_2$ anschließend $MnO_2$**

1 L der wässrigen, salzsauren Suspension des $TiO_2$ aus Beispiel 1 b wird mit NaOH auf einen pH-Wert von 9,0 gebracht und auf 80°C erhitzt. Anschließend werden zu der Suspension 52 mL Wasserglaslösung (entsprechend 384 g $SiO_2$ / L) zugegeben. Der pH- Wert steigt dabei auf ca. 10,6 an. Nach erfolgter Zugabe wird durch Zugabe von Schwefelsäure auf pH 6,5 abgesenkt und danach bei pH = 6,8 und 80°C 2 Stunden gerührt.

Anschließend werden bei 80°C 100mL einer $MnSO_4$-Lösung (5g Mn/L) bei konstantem pH-Wert (pH=7; Regelung durch Zugabe von NaOH) zugegeben. Die Suspension wird für weitere 30min gerührt. Das Produkt wird anschließend auf eine Leitfähigkeit kleiner 100 μS/cm gewaschen und getrocknet.

Vergleichsbeispiel

**6e: Beschichtung des nano-$TiO_2$ mit $SiO_2$ (Vergleich zu 6a)**

1 L der wässrigen, salzsauren Suspension des $TiO_2$ aus Beispiel 1 b wird mit NaOH auf einen pH-Wert von 6,5 gebracht und auf 80°C erhitzt. Anschließend werden zu der Suspension 52 mL Wasserglaslösung (entsprechend 384 g $SiO_2$ / L) bei konstantem pH-Wert (pH = 6,5 ± 0,5 ; Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 μS/cm gewaschen und getrocknet.

**Vergleichsbeispiel 7**

Die Mn-dotierte Titandioxidqualität Oxonica Optisol™ (aktuelles Marktprodukt der Firma Oxonica am Anmeldetag).

**Vergleichsbeispiel 8**

Rutil $TiO_2$ mit einer BET $100 m^2/g$ hergestellt, wie in WO 9960994, Example 3 beschrieben.

**Vergleichsbeispiel 9**

Degussa P25 (Rutil / Anatas) mit einer BET von 50m2/g.

**Beispiel 10: Charakterisierung der erfindungsgemäßen Produkte**

Die vorteilhaften Eigenschaften der erfindungsgemäßen Produkte können insbesondere mit den folgenden Methoden nachgewiesen werden:

**Methode 10a: Spinkonzentration von DPPH- bzw. TPA-haltigen Proben mittels continuous wave-EPR-Messung**

Die radikalreduzierende Wirkung der erfindungsgemäßen Produkte kann beispielsweise am 2,2-Diphenyl-1-picrylhydrazyl(DPPH)-Radikal oder am 2,2,5,5-Tetramethyl-Pyrrolin-1-yloxyl-3-acetylene (TPA)-Radikal nachgewiesen werden.

**Nomenklatur:**

Die Proben haben folgende Zuordnungen

| Proben-Nr. | Probenbeschreibung |
|---|---|
| 1 | $TiO_2$ Rutil Grundkörper ohne Coating |
| 2 | Mikro-Rutil ohne Fe-Dotierung und ohne $Al_2O_3$-Nachbehandlung mit 1 % Mn-Dotierung, 700 °C getempert |
| 3 | Mikro-Rutil mit 0,1 % Fe-Dotierung und 12 % $Al_2O_3$-Nachbehandlung |
| 4 | Mikro-Rutil mit 0,1 % Fe-Dotierung und 12 % $Al_2O_3$-Nachbehandlung |
| 5 | Mikro-Rutil ohne Fe-Dotierung und ohne $Al_2O_3$-Nachbehandlung |
| 6 | Mikro-Rutil mit 0,1 % Fe-Dotierung und 12 % $Al_2O_3$-Nachbehandlung mit 0,2 % Mn Auffällung |
| 8 | Mikro-Rutil mit 0,1 % Fe-Dotierung und 12 % $Al_2O_3$-Nachbehandlung mit 0,5 % Mn Auffällung 2 h bei 400 °C getempert |
| 9 | Mikro-Rutil mit 0,1 % Fe-Dotierung und 12 % $Al_2O_3$-Nachbehandlung mit 0,5 % Mn Auffällung 2 h bei 800 °C getempert |
| 10 | Optisol® von Oxonika; Batch 210 |
| 11 | Eusolex® T-2000 Standardware Charge K92002673; Fa. Merck |

**Messreihen:**

Reihe 0: reine Pigmentproben
Reihe 1: DPPH + Pigmentprobe nach dreiminütiger Reaktionszeit
Reihe 2: TPA + Pigmentprobe nach dreiminütiger Reaktionszeit
Reihe 3: DPPH + Pigmentprobe nach 96 Stunden Reaktionszeit

**Probenvorbereitung:**

Die Proben werden in perdeuteriertem Toluol suspendiert, so dass die Konzentration ca. 100 μM beträgt. Die DPPH und TPA-Lösungen werden ebenfalls 100 μM in perdeuterierten Toluol angesetzt. Für die DPPH-Messreihe werden jeweils 60 μL der Proben mit 60 μL DPPH-Lsg. in einem Eppendorf-Cup vereint und 100 μL in ein EPR-Probenröhrchen überführt. Die so erhaltene EPR-Probe wird 3 Minuten mit Argon gesättigt. Die DPPH-Referenzprobe wird analog mit 60 μL perdeuteriertem Toluol angesetzt. Für die TPA-Messreihe wird ebenso verfahren. Die EPR-Messungen an den DPPH-haltigen Proben werden nach 96 Stunden Reaktionszeit wiederholt.

**Methode:**

Unter identischen Messparametern ist die Intensität des erhaltenen continuous wave-EPR-Absorptionssignals von der Spinkonzentration der Probe ahängig. Das Integral des Absorptionssignals ist somit ein Maß für die Spinkonzentration der Probe. Aufgrund eines technischen Verstärkungsverfahrens wird allerdings die erste Ableitung des Absorptionsspektrums gemessen. Mittels einer Doppelintegration wird dann eine Aussage über die Spinkonzentration möglich. Es werden vergleichende Messungen zu einer Referenzprobe, die nur DPPH bzw. TPA enthält gegen Proben, die sowohl DPPH bzw. TPA als auch die Testsubstanzen enthalten gemessen. Die erhaltenen Doppelintegrale werden in Relation zueinander gesetzt. In jeder Messreihe wird die DPPH bzw. TPA-Referenzprobe dreimal gemessen und der arithmetische Mittelwert der Doppelintegrale benutzt.

**Messparameter:**

Die Apektren werden auf einem Standard E500 Bruker X-Band cw-Spektrometer bei Raumtemperatur unter folgenden Parametern aufgenommen:

**REINE PIGMENTPROBEN: (Reihe O)**

| | | | |
|---|---|---|---|
| T: | RT | freq.: | 9,4283GHz |
| field: | 480G - 6500G | delay: | 8s |
| points: | 4096 | scan: | 1 |
| microwave: | 23dB | receiver gain: | 60dB |
| mod. freq.: | 100kHz | mod. amp.: | 1G |
| time const.: | 81,92ms | conv.time: | 81,92ms |
| sweep time: | 335,54s | Q: | ~2500-2700 |

**DPPH + PIGMENT nach 3 min Reaktion: (Reihe 1)**

| | | | |
|---|---|---|---|
| T: | RT | freq.: | 9,4283GHz |
| field: | 480G - 6500G | delay: | 8s |
| points: | 4096 | scan: | 1 |
| microwave: | 23dB | receiver gain: | 60dB |
| mod. freq.: | 100kHz | mod. amp.: | 1G |
| time const.: | 81,92ms | conv.time: | 81,92ms |
| sweep time: | 335,54s | Q: | -2500-2700 |

**TPA + PIGMENT nach 3 min Reaktion: (Reihe 2)**

| | | | |
|---|---|---|---|
| T: | RT | freq.: | 9,4270GHz |
| field: | 3320G - 3390G | delay: | 3s |

(fortgesetzt)

| | | | |
|---|---|---|---|
| T: | RT | freq.: | 9,4270GHz |
| points: | 1024 | scan: | 10 |
| microwave: | 23dB | receiver gain: | 55dB |
| mod. freq.: | 100kHz | mod. amp.: | 1G |
| time const.: | 40,96ms | conv.time: | 40,96ms |
| sweep time: | 41,94s | Q: | ~2500-2700 |

**DPPH + Pigment nach 96 h Reaktion: (Reihe 3)**

| | | | |
|---|---|---|---|
| T: | RT | freq.: | 9,4266GHz |
| field: | 3325G - 3395G | delay: | 3s |
| points: | 1024 | scan: | 5 |
| microwave: | 23dB | receiver gain: | 55dB |
| mod. freq.: | 100kHz | mod. amp.: | 0,5G |
| time const.: | 81,92ms | conv.time: | 81,96ms |
| sweep time: | 83,89s | Q: | ~2500-2900 |

**Ergebnisse:**

Die Ergebnisse sind in der folgenden Tabelle bzw. Figur 1 zusammengefasst.

**Tabelle:** Spinkonzentration der untersuchten Proben

| Probe | Reihe 1 [%] | Fehler [%] | Reihe 2 [%] | Fehler [%] | Reihe 3 [%] | Fehler [%] |
|---|---|---|---|---|---|---|
| 1 | 79,68 | 20,88 | 85,98 | 22,53 | 71,58 | 18,78 |
| 2 | 53,00 | 13,89 | 103,20 | 27,03 | 110,9 | 29,05 |
| 3 | 36,33 | 9,52 | 89,13 | 23,35 | 73,70 | 19,31 |
| 4 | 62,15 | 16,28 | 93,79 | 24,57 | 97,88 | 25,64 |
| 5 | 50,18 | 13,15 | 87,27 | 22,86 | 99,46 | 26,06 |
| 6 | 25,06 | 6,56 | 91,85 | 24,07 | 67,66 | 17,73 |
| 8 | 39,43 | 10,33 | 99,23 | 26,00 | 44,22 | 11,58 |
| 9 | 37,43 | 9,81 | 93,14 | 24,40 | 96,41 | 25,26 |
| 10 | 53,96 | 14,14 | 96,22 | 25,21 | 107,30 | 28,12 |
| 11 | 52,55 | 13,77 | 88,92 | 23,30 | 116,60 | 30,56 |

**Methode 10b: Ranzimat-Test**

1,5g $TiO_2$ (s. Tabelle) werden mit 10,5g Sojaöl nicht stabilisiert (Lieferant Gustav Hees) für 15 Minuten mittels Magnetrührer bei RT gerührt. Von den so erhaltenen Suspensionen werden jeweils 4 g zur Messung verwendet. (Die Proben werden bis zum Messbeginn kühl und dunkel gelagert).

Die jeweiligen Proben werden in ein Reaktiongefäß in einen Rancimat 679 (Fa. Methrom) mit Auswerteeinheit und Schreiber überführt. (Reaktionstemperatur 120°C, Luftdruchfluß 15 Skalenteile.) Die Zersetzung des Sojaöls tritt je nach Zusatz ($TiO_2$) früher oder später auf. Die Ermittlung dieser Zersetzungsprodukte im Vergleich zu reinem Sojaöl erfolgt durch die Detektion entstehender Gase welche im Luftstrom ausgetrieben und im Wasser gelöst werden, mittels Leitfähigkeitsmessung.

Somit kann ein relativer Schutzfaktor gegenüber reinem Sojaöl ermittelt werden.

Relativer Schutzfaktor Sojaöl unstabilisiert = 1

Relativer Schutzfaktor < 1 bedeutet Substanz ist prooxidativ
Relativer Schutzfaktor > 1 bedeutet Substanz ist antioxidativ

| **Probe** | **Relative Stabilität im Vergleich zu unstabilisiertem Sojaöl** |
|---|---|
| Vergleichsbeispiel 2b | > 2,5 |
| Vergleichsbeispiel 6e | > 2,5 |
| Beispiel 7 | 0,35 |

**Methode 10c: Beurteilung der Produktfarbe**

Die Beurteilung kann an einem Pulverpressling oder in einer Formulierung erfolgen. Die Beurteilung erfolgt mit einem Farbmeßgerät (z. B. Macbeth Spectralphotometer Color Eye 7000) bei der Lichtart D65/10° ohne Glanz. Aus den Remissionen wurden die Farbabstände delta L* als Helligkeitsdifferenz der Probe im Bezug zu Beispiel 2b nach DIN 6174 berechnet.

| **Produktfarbe Pulverpressling (Delta L* nach DIN 6174)** | | |
|---|---|---|
| Titandioxidtyp | | |
| Vergleichsbeispiel 1b | | **+ 0,9** |
| Vergleichsbeispiel 2b | | **0 (Bezug)** |
| Beispiel 3a | | **-4,7** |
| Beispiel 3b | | **-6,1** |
| Beispiel 3c | | **-5,5** |
| Beispiel 3d | | **-7,2** |
| Beispiel 4a | | **-7,8** |
| Beispiel 4b | | **-9,8** |
| Beispiel 4c | | **-5,1** |
| Vergleichsbeispiel 5a | | **-0,3** |
| Beispiel 5b | | **-6,4** |
| Beispiel 5c | | **-8,9** |
| Beispiel 5d | | **-10,4** |
| Beispiel 6a | | **-3,7** |
| Vergleichsbeispiel 6e | | **+1,5** |
| Vergleichsbeispiel 7 | | **-14,7** |
| Vergleichsbeispiel 8 | | **-29,2** |
| Vergleichsbeispiel 9 | | **-30,7** |

Die Beurteilung der Produktfarbe in Formulierung erfolgt anhand folgender Formulierung:

| **Rohstoff (INCI)** | **Gew.%** |
|---|---|
| Titanium Dioxide, je Beispiel | 5 |
| PEG-100 Stearate, Glyceryl Stearate | 10 |
| PARAFFINUM LIQUIDUM (MINERAL OIL) | 25 |
| CETYL ALCOHOL | 2 |

(fortgesetzt)

| Rohstoff (INCI) | Gew.% |
|---|---|
| LANOLIN ANHYDROUS | 2 |
| BHT (and) GLYCERYL STEARATE (and) GLYCERYL OLEATE (and) ASCORBYL PALMITATE (and) CITRIC | 0,05 |
| ACID (and) PROPYLENE GLYCOL | |
| PROPYLPARABENE | 0,05 |
| SORBITOL | 3 |
| GLYCERIN | 2 |
| DISODIUM EDTA | 0,05 |
| METHYLPARABENE | 0,15 |
| AQUA (WATER) | ad 100 |

| Produktfarbe Formulierung (Delta L* nach DIN 6174) | | |
|---|---|---|
| Titandioxidtyp | | |
| Beispiel 3a | | **-5,2** |
| Beispiel 4a | | **-7,8** |
| Vergleichsbeispiel 7 | | **-19** |

Die Produktfarbe der Formulierung enthaltend die erfindungsgemäßen Produkte ist signifikant heller als die Vergleichsformulierung mit dem Marktprodukt von Vergleichsbeispiel 7. Die Ergebnisse sind auch in Figur 3 wiedergegeben.

**Methode 10d: UV Absorption eines Lackes**

**Herstellung der Lacke :**

Mahlpaste A:

Acrylat-Bindemittel (Macrynal SM 510 n;

| | |
|---|---|
| Fa. Cytec Surface Specialities) | 36,30 g |
| Xylol/Methoxyproylacetat 2:1 | 43,27 g |
| Produkt | 42,00 g |

Alle Zutaten werden bis auf 0,05g genau in eine 300mL PE-Weithalsflasche eingewogen und mit 300 g (+/-3g) Glasperlen (Durchmesser 2mm) versetzt.

Nach kurzem Durchschütteln von Hand (ca. 10 sec.) werden die Kautexflaschen in eine Schüttelmaschine eingespannt und 90 min geschüttelt.

Hilfsmittellösung B:

Dibutylzinn(IV)dilaurat (Mark DBTL; Fa. Cromption Vinyl Additives)

| | |
|---|---|
| (1%ig in Xylol) | 0,94 g |
| Diethylethanolamin | 1,68 g |
| Silikonöl L 050; Fa. Wacker Chemie | 4,41 g |
| Solvesso 100; Fa. Exxon Mobiole Chemical | 24,67 g |

(fortgesetzt)

| | |
|---|---|
| Xylol | 28,64 g |
| Methoxypropylacetat | 39,66 g |

Alle Zutaten werden homogenisiert.

Lackrezeptur

Acrylat-Bindemittel (Macrynal SM 510 n;

| | |
|---|---|
| Fa. Cytec Surface Specialities) | 39,90g |
| Mahlpaste A | 2,00g |
| Isocyanat-Bindemittel (Desmodur N 75; Fa. Bayer) | 17,21g |
| Hilfsmittellösung B | 19,74g |
| Xylol/Methoxyproylacetat 2:1 | 21,15g |

Für die Herstellung der Prüfanstriche wird die Mahlpaste A nach Mischen der anderen Komponenten der Lackrezeptur durch Rühren von Hand homogen zur Lackrezeptur zugemischt.

**Ermittlung der UV-Absorption (dekadischer Extinktionskoeffizient bei 300nm und 560nm)**

Die Lackrezeptur wird auf eine 60μm dicke Acetatfolie mit einem Spiralrakel (60μm) aufgezogen. Nach 5 min Ablüftzeit wird der Anstrich ca. 30 min bei 80°C forciert getrocknet. Die Extinktionsmessung erfolgt an einem UV-VIS Spektrometer bei 300nm bzw. 560nm.

Die Berechnung des dekadischen Extinktionskoeffizienten erfolgt wie folgt:

$C_{Probe}$ = Konzentration des untersuchten Musters aus Beispielen in g pro L (Feststoff)
$d_{Probe}$ = Trockenschichtdicke des auf Accetatfolie applizierten Lackes in cm
$E_{Probe}$ = Extinktion des Lackes mit Muster aus Beispielen
$E_{Klarlack}$ = Extinktion des Klarlackes
$\varepsilon_{Probe}$ = Dekadischer Extinktionskoeffizient (L g-1 cm-1) bei der entsprechenden Wellenlänge

$$\varepsilon_{Probe} = \frac{(E_{Probe} - E_{Klarlack})}{c_{Probe} \times d_{Probe}}$$

Der Wert bei 300 nm gibt die UV-Schutzleistung an. Je höher der Wert, desto besser der UV-Schutz.

Der Wert bei 560 nm gibt die Transparenz des Produktes an. Je niedriger der Wert ist, um so Transparenter ist das Produkt (eine Probe mit dem Wert von 0,6 ist 4x so transparent wie eine Probe mit einem Wert von 2,4).

| Probe | Dek. Extinktionskoeffizient bei 300 nm | Dek. Extinktionskoeffizient bei 560 nm |
|---|---|---|
| Beispiel 2b* | 47 | 0,63 |
| Beispiel 4a | 46 | 0,64 |
| Beispiel 8* | 34 | 2,38 |
| Beispiel 9* | 18 | 4,22 |
| * = Vergleichsbeispiel | | |

***Methode 10e: Transparenz im Sichtbaren (bei Wellenlänge 560nm)***

Die aufgelackte Mahlpaste A aus Methode 10d wird auf eine 60μm dicke Acetatfolie mit einem Spiralrakel (60 μm) aufgezogen. Nach 5 min Ablüftzeit wird der Anstrich ca. 30 min bei 80°C forciert getrocknet.

Die Transparenzmessung erfolgt mit einem UV-VIS Spektrometer bei. 560nm. Die Transparenz der Acetatfolie beschichtet mit einem Klarlack (ohne Probe) wird zu 100% gesetzt.

| Probe | Transparenz im Sichtbaren (bei Wellenlänge 560nm) |
|---|---|
| Beispiel 2b* | 96,3 % |
| Beispiel 4a | 96,2 % |
| Beispiel 8* | 86,7 % |
| Beispiel 9* | 77,6 % |
| * = Vergleichsbeispiel | |

***Methode 10f: In vitro SPF Bestimmung analog DIN 67502***

***Testrezeptur***

| Rohstoff (INCI) | Gew.% |
|---|---|
| PEG-100 STEARATE, GLYCERYL STEARATE | 10 |
| PARAFFINUM LIQUIDUM (MINERAL OIL) | 25 |
| CETYL ALCOHOL | 2 |
| LANOLIN ANHYDROUS | 2 |
| BHT (and) GLYCERYL STEARATE (and) GLYCERYL OLEATE (and) ASCORBYL PALMITATE (and) CITRIC ACID (and) PROPYLENE GLYCOL | 0,05 |
| PROPYLPARABENE | 0,05 |
| **Titandioxide gemäß Beispielen** | **5** |
| SORBITOL | 3 |
| GLYCERIN | 2 |
| DISODIUM EDTA | 0,05 |
| METHYLPARABENE | 0,15 |
| AQUA (WATER) | ad 100 |

| Testrezeptur enthaltend 5% Probe aus | In vitro SPF 0,75 mg/cm$^2$ auf PMMA |
|---|---|
| Beispiel 2b* | 8 |
| Beispiel 4a | 8 |
| Beispiel 7* | 3,5 |
| * = Vergleichsbeispiel | |

Methode 10g:

Die Menge an UV induzierten freien Radikalen (FR) wird in einer Schweinehautbiopsie bestimmt. Hierzu wird die Haut mit einem Haut-Radikalindikator markiert. Die FR reagieren mit der Probe (Emulsion mit $TiO_2$) und oxidieren

diese. Der verbleibende Gehalt an Radikalindikator -87-wird mittels ESR (Elektro-Spin-Resonanz-Spektroskopie) bestimmt. Nur freie Radikale aus dem Hautinneren oxidieren diesen Radikalindikator. Der RSF quantifiziert diese Menge an freien Radikalen und gibt eine Aussage darüber wie viel länger sich eine Individum in der Sonnen aufhalten kann, bei gleicher Radikalbildung (RSF 2 = Reduktion um 50% Freier Radikale).

Durchführung:

Die Formulierung wird auf die epidermale Seite einer Schweinehautbiopsie appliziert (2 mg/cm$^2$) und wird für 15 Minuten im Dunkeln gelagert.
Danach wird die Hautbiopsie mit der epidermalen Seite nach oben auf einem Filterpapier, welches mit einem Radikalindikator getränkt wurde, für 5 Minuten inkubiert.

- Es wird eine Hautstanze (4 mm Durchmesser) angefertigt und mit 1,2 MED bestrahlt.
- ESR-Messung : Durch die Reaktion des Radikalindikators mit der Probe vermindert sich das ESR Signal.
- Berechnung des RSF:

$$RSF = \frac{N_{freieRadikale} ungeschützt}{N_{freieRadikale} geschützt}$$

RSF Ergebnisse (nach 1,2 MED UV-Bestrahlung):

Placebo- O/W Formulierung ohne $TiO_2$ 1

| | |
|---|---|
| 5% Vergleichsbeispiel 9 | 2,8 |
| 5% Beispiel 2a zum Vergleich | 4,6 |
| 5% Vergleichsbeispiel 7 | 4,9 |
| 5% Beispiel 3a | 6,4 |
| 5% Beispiel 3b | 7,4 (= 84% Reduktion der freien Radikale im Vegleich zu Placebo). |

Hieraus kann man erkennen, dass die erfindungsgemäßen Beispiele 3a/4a ein starkes radikalfangedes Potential aufweisen.

**Methode 10h:**

Wirksamkeitstest in vivo gegen photo-oxidativen Stress der Haut, induziert durch UVA-Bestrahlung - der "β-Carotin-Test".
Getestet wird die in-vivo Wirksamkeit der erfindungsgemäßen Produkte in der Testformulierung, wie in der Methode 10c und g beschrieben.
Die Formulierung wird an 10 gesunden Menschen getestet, sowohl männlich wie weiblich mit einem Alter über 18 Jahren. Die Auftragung von 2 mg/cm$^2$ Formulierung erfolgt auf die innere Seite des Unterarms in einem Gebiet von 35 cm$^2$. Nach 20 Minuten werden 80 μl einer β-Carotin-Lösung appliziert (5mg/100ml in n-Hexan oder einer übersättigten und filtrierten Lösung in n-Hexan) und 3 Minuten später beginnt die UVA-Bestrahlung (10 J/cm$^2$). Die Messung der Verfärbung erfolgt über die Bestimmung des b*-Wertes im Vergleich zu einem durch β-Carotin verfärbten Testgebiets ohne Auftragung der Formulierung, wie zuvor beschrieben. Die Messung des b*-Wertes erfolgt über ein Chromameter.

**Chromameter-Messung**

Die Farbbewertung wurde mit Hilfe des *Minolta Chromameters CR-300* durchgeführt. Chromameter sind Farbmessgeräte, die ursprünglich für den Bereich der Farbenherstellung und Farbenverarbeitung in der Industrie eingesetzt worden sind. Mit Hilfe dieser Geräte lassen sich unterschiedliche Farbnuancen exakt und numerisch darstellen [60]. Als Farbsystem eignet sich das von der Commission Internationale de l'Eclairage (CIE) 1976 entwickelte Farbmaßsystem

[6], das ähnlich wie die Farbverarbeitung des menschlichen Auges auf dem Dreibereichsverfahren beruht mit den Primärfarben Rot, Grün und Blau. Jeder Farbton lässt sich exakt durch eine vektorielle Darstellung in einem dreidimensionalen System mit den Koordinaten L* (Helligkeit), a* (rot-grün) und b* (gelb-blau) bestimmen.

Darüber hinaus ist das L*a*b*-System den visuell empfundenen Farbabständen am ähnlichsten. Aufgrund dieser parallelen Grundzüge zum physiologischen Farbverarbeitungssystem lassen sich diese industriell eingesetzten Farbmessgeräte ebenfalls in der Dermatologie zur Beurteilung des Hautkolorits anwenden und wurden auch schon in der Vergangenheit erfolgreich zur Messung von kutanen Farbunterschieden eingesetzt [5], [11], [12], [14], [17], [18], [20], [23], [47].

5. Chan, S.Y., Li Wan Po, A. Quantitative evaluation of drug-induced erythema by using a tristimulus colour analyzer: experimental design and data analysis. Skin. Pharmacol. 6 (1993) 298-312

11. Eckhardt, L., Mayer, J.A., Creech, L., Johnston, M.R., Lui, K.J., Sallis, J.F., Elder, J.P. Assessing children's ultraviolet radiation exposure: the potential usefulness of a colorimeter. Am. J. Public Health 86 (1996) 1802-1804

12. Fluhr, J.W., Pfisterer, S., Gloor, M. Direct comparison of skin physiology in children and adults with bioengineering methods. Pediatr. Dermatol. 17 (2000) 436-439

14. Fullerton, A., Benefeldt, E., Petersen, J.R., Jensen, S.B., Serup, J. The calcipotrion dose-irritation relationship: 48 hour occlusive testing in healthy volunteers using Finn Chambers. Br. J. Dermatol. 138 (1998) 259-265

17. Garigue, J., Marguery, M.C., Malmary, M.F., el Sayed, F., Bazex, J. Measurement of actinic erythema in healthy subjects and in subjects with polymorphous light eruption using a tristimulus colorimeter. Dermatology 190 (1995) 31-34

18. Gassmueller, J., Maas-Irslinger, R., Rippke, F., Tausch, I. Antiinflammatorische Wirksamkeit magistraler Rezepturen mit Glukokortikosteroiden in Eucerinum-Fertiggrundlagen im Vergleich zu Fertigpräparaten im UVB-Erythemtest. Zeitschrift für Hautkrankheiten, H+G 6 (1998) 364-370

20. Guarrera, M., Brusati, C., Rebora, A. Topical metronidazole does not abate uvb-induced erythema. Dermatology 203 (2001) 121-123

23. Henry, F., Fumal, I., Pierard, G.E. Postural skin colour changes during the corticosteroid blanching assay. Skin. Pharmacol. Appl. Skin. Physiol. 12 (1999) 199-210

47. Seitz, J.C., Whitmore, C.G. Measurement of erythema and tanning responses in human skin using a tristimulus colorimeter. Dermatologica 177 (1988) 70-75

60. Tronnier, M., Schulz, R., Wolff, H.H. Colorimetrische Erythemmessung nach UVB-Bestrahlung an gesunder Haut in Abhängigkeit von unterschiedlicher Vorbehandlung. Akt. Dermatol. 18 (1992) 183-186

Die Ergebnisse werden als Rate der Inhibierung gegenüber unbehandeltem Areal ausgedrückt. Der Farbindex in % korrespondiert direkt mit der Wirksamkeit gegen freie Radikale.

$$Farb-Index = 100 - \left( \frac{\mathrm{Pr}\,obe_{\mathrm{eingefärbt}} - \mathrm{Pr}\,obe_{\mathrm{bestrahlt}}}{Kontrolle_{\mathrm{eingefärbt}} - Kontrolle_{\mathrm{bestrahlt}}} \times 100 \right)$$

eingefärbt bedeutet die ermittelte Farbe nach Färbung mit beta-Carotin bestrahlt bedeutet die ermittelte Farbe nach Einfärbung & Bestrahlung.

Ergebnisse:

Beispiel 2b* - 47 %
Beispiel 3a - 48 %
Beispiel 4a - 61 %

Beispiel 9* - 17%.

Die Ergebnisse sind grafisch in Figur 2 dargestellt. * = Vergleichsbeispiel

Die Ergebnisse belegen die hervorragende photoprotektive Wirksamkeit der erfindungsgemäßen Produkte in vivo.

**Rezepturbeispiel 1: Sonnenschutz Softcreme (O/W)**

| **Rohstoff (INCI)** | **Gew.%** |
|---|---|
| **A** | |
| Produkt aus Beispiel 3a | 3,00 |
| Steareth-10, Steareth-7, Stearyl alcohol | 2,00 |
| Glyceryl stearate, Ceteth-20 | 2,00 |
| Glyceryl stearate | 3,00 |
| Microwax | 1,00 |
| Oleyl oleate | 6,00 |
| Cetearyl octanoate | 14,00 |
| Caprylic/capric triglyceride | 4,00 |
| Propylparaben | 0,05 |
| **B** | |
| Propylene glycol | 4,00 |
| Allantoin | 0,20 |
| Wasser | 60,60 |
| Methylparaben | 0,15 |

**Herstellung:**

Phase A und Phase B auf 80 °C erhitzen. Phase B unter Rühren langsam zu Phase A geben, homogenisieren und unter Rühren abkühlen.

**Rezepturbeispiel 2: Sonnenschutz Spray-Lotion (O/W)**

| **Rohstoff (INCI)** | % |
|---|---|
| **A** | |
| Ethylhexyl methoxycinnamate, BHT | 5,00 |
| Produkt aus Beispiel 3b | 4,00 |
| Glyceryl stearate, cetyl alcohol, PEG-75 stearate, ceteth-20, steareth-20 | 3,30 |
| PPG-1-PEG-9 lauryl glycol ether | 0,50 |
| Diisostearoyl) trimethylolpropane | 1,50 |
| Siloxy silicate | |
| $C_{12-15}$ alkyl benzoate | 3,00 |
| Dioctyl adipate | 4,00 |
| Dimethicone | 2,00 |
| **B** | |
| Dimethicone copolyol phosphate | 2,50 |
| Butylene glycol | 2,50 |
| Wasser | 70,50 |
| **C** | |
| PPG-1 Trideceth-6, polyquaternium-37, propylene | |

(fortgesetzt)

| | |
|---|---|
| **C** | |
| glycol dicaprylate/dicaprate | 0,47 |
| **D** | |
| Propylene glycol, DMMDM hydantoin, methylparaben, propylparaben | 0,73 |

**Herstellung:**

Phase A bis auf das Titandioxid zusammengeben und auf 60 °C erhitzen. Titandioxid langsam in die geschmolzene Ölphase einarbeiten. Phase B auf 60 °C erhitzen, dann Phase C unter Rühren eindispergieren. Phase A unter hohem Energieeintrag in die Phase B/C einrühren. Unter Rühren abkühlen, und bei 40 °C Phase D zugeben. Homogenisieren und unter Rühren auf 25 °C abkühlen.

| **Rohstoff (INCI)** | **%** |
|---|---|
| **A** | |
| Produkt aus Beispiel 3c | 10,00 |
| Steareth-10, steareth-7, stearyl alcohol | 3,00 |
| Glyceryl stearate, ceteth-20 | 3,00 |
| Glyceryl stearate | 3,00 |
| Microwax | 1,00 |
| Oleyl oleate | 4,00 |
| Cetearyl octanoate | 10,50 |
| Caprylic/capric triglyceride | 4,00 |
| Propylparaben | 0,05 |
| **B** | |
| Propylene glycol | 4,00 |
| Allantoin | 0,20 |
| Wasser | 57,10 |
| Methylparaben | 0,15 |

**Herstellung:**

Phase A und B auf 80 °C erhitzen. Phase B unter Rühren langsam zu Phase A geben, homogenisieren und unter Rühren abkühlen.

**Rezepturbeispiel 4: Sonnenschutzlotion (O/W)**

| **Rohstoff (INCI)** | **%** |
|---|---|
| **A** | |
| Ethylhexyl methoxycinnamate, BHT | 6,00 |
| Butyl methoxydibenzoylmethane | 1,00 |
| Polyglyceryl-3 methylglucose distearate | 4,00 |
| Ethylhexyl stearate | 8,00 |
| Cetearyl isononanoate | 2,00 |
| PVP/eicosene copolymer | 1,00 |
| Tocopheryl acetate | 1,00 |
| **B** | |
| Xanthan gum | 0,30 |
| Sodium cetearyl sulfate | 1,00 |
| Glycerin | 5,00 |
| Wasser | 65,70 |

(fortgesetzt)

| | |
|---|---|
| **C** | |
| Produkt aus Beispiel 4a | 4,00 |
| **D** | |
| Phenoxyethanol, butylparaben, ethylparaben, propylparaben, methylparaben | 1,00 |

**Herstellung:**

Phase A auf 80 °C erhitzen. Das Keltrol der Phase B im Wasser vorquellen, dann die restlichen Rohstoffe zugeben und auf 80 °C erhitzen. Phase A zu Phase B gegen und 2 Min. homogenisieren (Stabmixer): unter Rühren abkühlen und bei 35 °C Phase C zufügen. Nochmals 1 Min. homogenisieren (Stabmixer). Auf Raumtemperatur abkühlen und Phase D einrühren.

**Rezepturbeispiel 5: Sonnenschutzlotion (O/W)**

| Rohstoff (INCI) | % |
|---|---|
| **A** | |
| Produkt aus Beispiel 3d | 5,00 |
| Ethylhexyl methoxycinnamate, BHT | 5,00 |
| Glyceryl stearate, cetyl alcohol, PEG-75 stearate, ceteth-20, steareth-20 | 3,30 |
| PPG-1-PEG-9 lauryl glycol ether | 0,50 |
| Diisostearoyl trimethylolpropane siloxy silicate | 1,50 |
| C12-15 Alkyl benzoate | 3,00 |
| Dioctyl adipate | 4,00 |
| Dimethicone | 2,00 |
| **B** | |
| Ectoin | 0,10 |
| Allantoin | 0,20 |
| Dimethicone copolyol phosphate | 2,50 |
| Butylene glycol | 2,50 |
| Wasser | 68,90 |
| **C** | |
| PPG-1 trideceth-6, polyquaternium-37, propylene glycol dicaprylate/dicaprate | 0,47 |
| D | |
| Propylene glycol, DMMDM hydantoin, ehtylparaben | 0,73 |
| Parfum | 0,30 |

**Herstellung:**

Phase A bis auf das Titandioxid zusammen geben und auf 60 °C erhitzen. Titandioxid langsam in die geschmolzene Ölphase einarbeiten. Phase B auf 60 °C erhitzen, dann Phase C unter Rühren eindispergieren. Phase A unter kräftigem Rühren in die Phase B/C einrühren. Unter Rühren abkühlen und bei 40 °C Phase D zugeben. Homogenisieren und unter Rürhen auf 25 °C abkühlen.

**Rezepturbeispiel 6: Sonnenschutzlotion (O/W)**

| Rohstoff (INCI) | % |
|---|---|
| **A** | |
| Produkt aus Beispiel 4a bzw. 4b | 5,00 |
| Butylmethoxy dibenzoylmethane | 3,00 |
| Steareth-10, steareth-7, stearyl alcohol | 3,00 |
| Glyceryl stearate, ceteth-20 | 3,00 |
| Glyceryl stearate | 3,00 |
| Microwax | 1,00 |
| Oleyl oleate | 4,43 |
| Cetearyl octanoate | 11,64 |
| Caprylic/capric triglyceride | 4,43 |
| Propylparaben | 0,05 |
| **B** | |
| Propylene glycol | 4,00 |
| Allantoin | 0,20 |
| Wasser | 57,10 |
| Methylparaben | 0,15 |

**Herstellung:**

Phase A und B auf 80 °C erhitzen. Phase B unter Rühren langsam zu Phase A geben, homogenisieren und unter Rühren abkühlen.

**Rezepturbeispiel 7: Sonnenschutzlotion (O/W) SPF 10 (Sun Protection Factor, Colipa-Methode mit 10 Probanden)**

| | |
|---|---|
| **A** | |
| Steareth-10, steareth-7, stearyl alcohol | 3,00 |
| Glyceryl stearate, ceteth-20 | 3,00 |
| Cetearyl octanoate | 15,50 |
| Glyceryl stearate | 3,00 |
| Oleyl oleate | 7,00 |
| Microwax | 1,00 |
| Caprylic/capric triglyceride | 6,00 |
| Propylparaben | 0,05 |
| **B** | |
| 33%-ige wässrige Dispersion des Produktes aus Beispiel 5a | 16,70 |
| Propylene glycol | 4,00 |
| Allantoin | 0,20 |
| Wasser | 40,40 |
| Methylparaben | 0,15 |

**Herstellung:**

Phase A auf 75 °C und Phase B auf 80 °C erhitzen. Phase B langsam in Phase A einrühren. Homogenisieren und unter Rühren abkühlen.

**Rezepturbeispiel 8: Sonnenschutz Spray-Lotion (O/W)**

| | |
|---|---|
| **A** | |
| Produkt aus Beispiel 5d | 5,00 |
| Ethylhexyl methoxycinnamate, BHT | 7,50 |
| Benzophenone-3 | 2,50 |
| PEG-100 stearate, glyceryl stearate | 2,80 |
| PPG-1-PEG-9 lauryl glycol ether | 0,40 |
| Dicapryl ether | 4,50 |
| Steareth-10 | 0,50 |
| Stearyl alcohol | 0,60 |
| Dimethicone | 2,00 |
| **B** | |
| Dimethicone copolyol phosphate | 2,50 |
| Chitosan glycolate | 2,00 |
| Glycerin | 2,50 |
| Wasser | 66,10 |
| **C** | |
| PPG-1 trideceth-6, polyquaternium-37, propylene glycol dicaprylate/dicaprate | 0,40 |
| **D** | |
| Propylene glycol, DMMDM hydantoin, methylparaben, propylparaben | 0,70 |

**Herstellung:**

Phase A bis auf das Titandioxid zusammen geben und auf 60 °C erhitzen. Titandioxid langsam in die geschmolzene Ölphase einarbeiten. Phase B-1 auf 60 °C erhitzen, dann Phase B-2 unter Rühren eindispergieren. Phase A unter hohem Energieeintrag in die Phase B einrühren. Unter Rühren abkühlen, und bei 40 °C Phase C zugeben. Homogenisieren und unter Rühren auf 25 °C abkühlen.

**Rezepturbeispiel 9: Sonnenschutz Creme, hoher SPF, wasserfest (O/W)**

| | |
|---|---|
| **A** | |
| Wasser | 38,30 |
| Glycerin | 3,00 |
| Pentylene glycol | 3,00 |
| PVP/hexadedecene copolymer | 1,00 |
| Sodium cetearyl sulfate | 1,00 |
| Xanthan gum | 0,20 |
| **B** | |
| Glyceryl stearate, cetearyl alcohol, sodium stearoyl lactylate, tocopherol | 5,00 |
| Tri-C12-13 alkyl citrate | 3,50 |
| Isopropylphtalimide, butylphtalide | 5,00 |
| Caprylic/capric triglyceride | 2,50 |
| C12-15 alkyl benzoate | 2,00 |
| Cyclomethicone | 0,80 |
| Tocopheryl acetate | 1,00 |
| Butyl methoxydibenzoylmethane | 1,00 |
| Benzophenone-3 | 2,00 |
| Produkt aus Beispiel 4c | 4,00 |
| **C** | |
| Wasser, ethylhexyl methoxycinnamate, silica, PVP, | |

(fortgesetzt)

| | |
|---|---|
| C | |
| chlorphenesin, BHT (Eusolex UV Pearl OMC) | 20,00 |
| D | |
| Carbomer | 0,15 |
| Wasser | 4,85 |
| E | |
| Sodium hydroxide | 0,90 |
| F | |
| Phenoxyethanol, butylparaben, ethylparaben, propylparaben, methylparaben | 0,50 |
| Parfum | 0,30 |

**Herstellung:**

Phasen A und B getrennt voneinander auf 80 °C erhitzen. Phase B mit dem Thurrax homogenisieren, bis das Pigment schön benetzt ist. Phase B zu Phase A geben und 2 Min. homogenisieren. Auf 35 °C abkühlen, Phase C zufügen und 30 Sek. homogenisieren. Phase D zufügen und 30 Sek. homogenisieren. Phase E unterrühren und mit Phase F neutralisieren und homogenisieren bis eine zufriedenstellende Pigmentverteilung erreicht ist (mikroskopische Kontrolle!). Auf Raumtemperatur abkühlen, entlüften und Phase G einrühren.

**Rezepturbeispiel 10: Sun Protection Lotion (PEG-free) in vitro SPF 12 ± 2**

| Rohstoff (INCI) | % |
|---|---|
| **A** | |
| C12-15 Alkyl benzoate | 3,00 |
| Decyl cocoate | 4,00 |
| Ethylhexylpalmitate | 3,00 |
| Glyceryl stearate | 0,50 |
| Stearic acid | 0,50 |
| Tocopheryl acetate | 0,50 |
| **B** | |
| Cetearyl glucoside | 1,50 |
| Propylene glycol | 2,00 |
| Glycerin | 1,00 |
| Wasser | 76,80 |
| **C** | |
| Produkt aus Beispiel 4a | 5,00 |
| **D** | |
| Carbomer | 0,20 |
| Paraffinum liquidum (mineral oli) | 0,80 |
| **E** | |
| Sodium hydroxide | 0,50 |
| **F** | |
| Propylene glycol, diazolidinyl urea, methylparaben, propylparaben | 0,50 |
| Parfum | 0,20 |

**Herstellung:**

Phase A und Phase B getrennt auf 80 °C erhitzen. Phase A unter Rühren zu Phase B geben. Bei 40 °C Phase C unter Rühren in die Emulsion eintragen und homogenisieren bis zur optimalen Pigmentverteilung. Bei 35 °C Phase

D zugeben und nochmals kurz homogenisieren. Phase E zugeben, pH-Wert kontrollieren und nochmals kurz homogenisieren. Phase F zugeben und kalt rühren.

**Rezepturbeispiel 11: W/O Sonnenschutzlotion mit anorganischem Filter, in vitro SPF (Diffey-Methode) 8,7 ± 1,6, UVA-PF 4,4 ± 0,5**

| **Rohstoff (INCI)** | **%** |
|---|---|
| **A** | |
| Cetyl PEG/PPG-10/1 Dimethicone | 2,50 |
| Stearoxy dimethicone | 0,25 |
| Ethylhexyl stearate | 12,75 |
| Ethylhexyl palmitate | 8,00 |
| Isohexadecane | 7,00 |
| hydrogenate castor oil | 0,50 |
| Ceresin (microcrystalline wax) | 1,00 |
| **B** | |
| Produkt aus Beispiel 5b | 5,00 |
| **C** | |
| Wasser | 62,00 |
| Sodium chloride | 0,50 |
| Propylene glycol, diazolidinyl usrea, methylparaben, propylparaben | 0,50 |

**Herstellung:**

Phase A auf 80 °C erhitzen. Das Titandioxid (Phase B) sorgfältig in die heiße Ölphase einarbeiten. Phase C langsam unter Rühren (500 upm, (Mig-Rührer) zu Phase A/B geben. Bei 1600 upm für 2 Minuten homogenisieren. Unter Rühren ca. 300 upm) auf ca. 40 °C abkühlen und nochmals für 2 Minuten bei 1600 upm homogenisieren.

**Rezepturbeispiel 12 Anti Ageing Cremegel- Zellschutz Intensiv (O/W)**

| **Rohstoff** | **INCI** | **[%]** |
|---|---|---|
| **A** | | |
| RonaCare® Ectoin | ECTOIN | 2,00 |
| Titriplex® III | DISODIUM EDTA | 0,10 |
| Wasser, demineralisiert | AQUA (WATER) | ad 100 |
| **B** | | |
| **Produkt aus Beispiel 5d** | | **3,00** |
| Eusolex® OS | ETHYLHEXYL SALICYLATE | 3,00 |
| Eusolex® OCR | OCTOCRYLENE | 3,00 |
| **RonaCare AP®** | **Hydroxy Dimethoxybenzyl Malonate** | **1,00** |
| Eusolex® 9020 | BUTYL METHOXYDIBENZOYLMETHANE | 0,50 |
| Cegesoft C 24 | ETHYLHEXYL PALMITATE | 4,00 |
| Sepiplus 400 | POLYSORBATE 20, POLYACRYLATE 13, POLYISOBUTENE | 2,00 |
| **C** | | |
| Phenonip | PHENOXYETHANOL, BUTYLPARABEN, ETHYLPARABEN, PROPYLPARABEN, METHYLPARABEN | 0,70 |

**Rezepturbeispiel 13**

Im Folgenden werden beispielhaft Rezepturen für kosmetische Zubereitungen angegeben, die in gleicher Weise

mit Titandioxid nach Beispiel 3a, 3b, 3c, 3d, 4a, 4b, 4c, 5c, 5d, 5e, 5f, 5g oder 5h enthalten werden (In der Tabelle jewels als Titanium dioxide bezeichnet). Im Übrigen sind die INCI-Bezeichnungen der handelsüblichen Verbindungen angegeben.

UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im Handel unter der Bezeichnung Eusolex®UV Pearls™OMC von der Merck KGaA, Darmstadt erhältlich.

Die anderen in den Tabellen angegebenen UV-Pearls sind jeweils analog zusammengesetzt, wobei OMC gegen die angegebenen UV-Filter ausgetauscht wurde.

## Tabelle 1: W/O-Emulsionen (Zahlen in Gew.%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | 3 | 2 | 5 | 10 | 7 | 4 | 15 | 1 | 3 | 3 |
| Butylmethoxydibenzoyl-methane | 5 | 3 | 2 | 1 | 2 | | | | 1 | 1 |
| Zinc oxide | | | | | | | | 5 | 2 | |
| UV-Pearl, OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

**Tabelle 1 (Fortsetzung)**

| | 1-11 | 1-12 | 1-13 | 1-14 | 1-15 | 1-16 | 1-17 | 1-18 |
|---|---|---|---|---|---|---|---|---|
| Titanium dioxide | 3 | 5 | 2 | 4 | 3 | 1 | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Dihydroxyavcetone | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |

| | | | | | | | | |
|---|---|---|---|---|---|---|---|---|
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| Water | ad 100 | ad 100 | ad 100 | ad 100 | ad 100 | ad 100 | ad 100 | ad 100 |

**Tabelle 1 (Fortsetzung)**

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | 1 | 2 | 5 | 1 | 3 | 4 | 5 | 2 | 3 | 3 | 3 |
| Benzylidene malonate polysiloxane | | | | 1 | | | | | 1 | 1 | |
| Methylene Bis-Benz-triazolyl Tetramethyl-butylphenol | | | | | | 1 | 2 | 1 | | | 1 |
| Zinc oxide | | | | | | | | 5 | 2 | | |
| UV-Pearl OMC | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl , OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| Butylmethoxydibenzoyl methane | | | | | | | | | | | 2 |

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

**Tabelle 2: O/W-Emulsionen, Zahlen in Gew.%**

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | 3 | 2 | 5 | 2 | 5 | 2 | 5 | 2 | 5 | 3 |
| Methylene Bis-Benztriazolyl Tetramethylbutylphenol | | | | | | 1 | 2 | 1 | | |
| Butylmethoxydibenzoyl-methane | 3 | 3 | 3 | 3 | 3 | 3 | 3 | 3 | 3 | 3 |
| 2-(4-Hydroxy-3,5-dimethoxy-benzyliden)-malonsäure-bis-(2-ethyl-hexyl)ester | 1 | 5 | 4 | | 6 | | 7 | | 2 | 1 |
| 4-Methylbenzyliden Camphor | 2 | | 3 | | 4 | | 3 | | 2 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

**Tabelle 2 (Fortsetzung)**

| | 2-11 | 2-12 | 2-13 | 2-14 | 2-15 | 2-16 | 2-17 | 2-18 |
|---|---|---|---|---|---|---|---|---|
| Titanium dioxide | 3 | 1 | 2 | 5 | 4 | 3 | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Butylmethoxydibenzoyl-methane | 3 | 3 | 3 | 3 | 3 | 3 | 3 | 3 |
| 2-(4-Hydroxy-3,5-dimethoxy-benzyliden)-malonsäure-bis-(2-ethyl-hexyl)ester | 1 | 5 | 4 | | 6 | | 7 | |

| | 2-11 | 2-12 | 2-13 | 2-14 | 2-15 | 2-16 | 2-17 | 2-18 |
|---|---|---|---|---|---|---|---|---|
| Zinc oxide | | | 2 | | | | | |
| UV-Pearl, OMC | 15 | 15 | 15 | 30 | 30 | 30 | 15 | 15 |
| 4-Methylbenzyliden Camphor | | | | 3 | | | | |
| *Phenylbenzimidazole* Sulfonic Acid | | | | | 4 | | | |
| Stearyl Alcohol (and) Steareth-7 (and) Steareth-10 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

**Tabelle 2 (Fortsetzung)**

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Titanium dioxide | 10 | 5 | 7 | 8 | 2 | 1 | 3 | 3 | 6 | 2 |
| Benzylidene malonate polysiloxane | 1 | 2 | | | | 1 | 1 | | 1 | 0,5 |
| Butylmethoxydibenzoyl-methane | 3 | 3 | 3 | 3 | 3 | 3 | 3 | 3 | 3 | 3 |
| Octocrylene | 1 | 5 | 4 | | 6 | | 7 | | 2 | 1 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl, OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

**Tabelle 3: Gele, Zahlen in Gew.%**

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | 5 | 2 | 5 | 1 | 1 | 1 | 1 | 1 | 3 | 3 |
| Butylmethoxydibenzoyl-methane | 2 | 2 | 2 | 2 | 2 | 2 | 2 | 2 | 2 | 2 |
| Dihydroxyacetone | 1 | 5 | 4 | | 6 | | 7 | | 2 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |

| | 3-1 | 3-2 | 3-3 | 3-4 | 3-5 | 3-6 | 3-7 | 3-8 | 3-9 | 3-10 |
|---|---|---|---|---|---|---|---|---|---|---|
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

## Tabelle 3 (Fortsetzung)

| | 3-11 | 3-12 | 3-13 |
|---|---|---|---|
| a = aqueaous gel | | | |
| Titanium dioxide | 3 | 1 | 2 |
| Benzylidene malonate polysiloxane | | 1 | 0,5 |
| Methylene Bis-Benztriazolyl Tetramethylbutylphenol | 1 | 1 | 0,5 |
| Butylmethoxydibenzoylmethane | 2 | 2 | 2 |
| 2-(4-Hydroxy-3,5-dimethoxy-benzyliden)-malonsäure-bis-(2-ethyl-hexyl)ester | 1 | 5 | 4 |
| Zinc oxide | | | 2 |
| UV-Pearl , Ethylhexyl Mehtoxycinnamat | 15 | 15 | 15 |
| Prunus Dulcis | 5 | 5 | 5 |
| Tocopheryl Acetate | 0,5 | 0,5 | 0,5 |
| Caprylic/Capric Triglyceride | 3 | 3 | 3 |
| Octyldodecanol | 2 | 2 | 2 |
| Decyl Oleate | 2 | 2 | 2 |
| PEG-8 (and) Tocopherol (and) Ascorbyl Palmitate (and) Ascorbic Acid (and) Citric Acid | 0,05 | 0,05 | 0,05 |
| Sorbitol | 4 | 4 | 4 |
| Polyacrylamide (and) C13-14 Isoparaffin (and) Laureth-7 | 3 | 3 | 3 |
| Carbomer | | | |
| Propylparabene | 0,05 | 0,05 | 0,05 |
| Methylparabene | 0,15 | 0,15 | 0,15 |
| Allantoin | | | |
| Tromethamine | | | |
| Water | ad 100 | ad 100 | ad 100 |

## Tabelle 3 (Fortsetzung)

| | 3-14 | 3-15 | 3-16 | 3-17 | 3-18 | 3-19 | 3-20 | 3-21 |
|---|---|---|---|---|---|---|---|---|
| Titanium dioxide | 1 | 5 | 3 | 1 | 2 | 8 | 12 | 1 |
| Butylmethoxydibenzoylmethane | 2 | 2 | 2 | 2 | 2 | 2 | 2 | 2 |
| UV-Pearl , OMC | 15 | 10 | | 10 | 10 | 10 | 15 | 10 |
| UV-Pearl , OCR | | | 10 | | | | | |
| UV-Pearl , OMC, Methylene Bis-Benztriazolyl Tetramethylbutylphenol | | 7 | | 6 | | | | |
| UV-Pearl, Ethylhexyl salicylate, Butylmethoxydibenzoylmethane | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

**Rezepturbeispiel 17: Anti-Ageing Cremegel (O/W)**

| Rohstoff | INCI | [%] |
|---|---|---|
| A | | |
| RonaCare AP | HYDROXY DIMETHOXYBENZYL MALONATE | 4,00 |
| Eusolex OCR | Octocrylene | 4,00 |
| Eusolex 9020 | Butyl Methoxydibenzoylmethane | 2,00 |
| Tegin | Glyceryl Stearate SE | 1,50 |
| Montanov S | Coco Glucoside; Coconut Alcohol | 1,50 |

(fortgesetzt)

| Rohstoff | INCI | [%] |
|---|---|---|
| A | | |
| Dow Corning 246 | Cyclohexasiloxane | 5,00 |
| Cetiol A | Hexyl Laurate | 5,00 |
| Titanium Dioxide | | 1.00 |
| B | | |
| Wasser, demineralisiert | AQUA (WATER) | ad 100 |
| Glycerin (87%) | Glycerin, Aqua | |
| Konservierungsmittel | Preservative | q.s. |
| Titriplex II | Disodium EDTA | 0,10 |
| C | | |
| Simulgel EPG | Sodium polyacrylate (and) Sodium acryloyldimethyltautrate copolymer (and) polyisobutene (and) caprylyl capryl glucoside | 1,20 |
| Keltrol CG-SFT | Xanthan Gum | 0,20 |
| Dow Corning 245 | Cyclomethicone | 5,00 |
| D | | |
| Dow Corning HMW 220 | Divinyldimethicone/Dimethicone Copolymer (and) C12-13 Pareth-3 (and) C12-13 Pareth-23 | 3,00 |
| Parfüm | Fragrance | q.s. |

**Rezepturbeispiel 18: Environmental Block Cremegel (O/W)**

| Rohstoff | INCI | [%] |
|---|---|---|
| **A** | | |
| **RonaCare AP** | **HYDROXY DIMETHOXYBENZYL MALONATE** | **2,00** |
| Montanov 202 | ARACHIDYL ALCOHOL, BEHENYL ALCOHOL, ARACHIDYLGLUCOSIDE | 3,00 |
| X-Tend 226 | PHENYLETHYL BENZOATE | 8,00 |
| Pelemol BIP | ISOPROPYLPHTALIMIDE, BUTYLPHTALIDE | 2,00 |
| Permethyl 102A | ISOEICOSANE | 1,00 |
| Silkflo 366 NF | HYDROGENATED POLYDECENE | 1,00 |
| Eusolex 9020 | BUTYL METHOXYDIBENZOYLMETHANE | 1,00 |
| Titandioxid aus Beispiel 3a | | 1,00 |
| B | | |
| Glycerin (87% reinst) | GLYCERIN | 3,00 |
| Wasser, demineralisiert | AQUA (WATER) | ad 100 |
| C | | |
| Simulgel NS | HYDROXYETHYL ACRYLATE/SODIUM ACRYLOYLDIMETHYLTAURATE COPOLY-MER, SQUALANE, POLYSORBATE 60 | 2,05 |
| **D** | | |
| Germaben II | PROPYLENE GLYCOL, DIAZOLIDINYL UREA, METHYLPARABEN, PROPYLPARABEN | 0,75 |
| NaOH (10%) | Aqua, Sodium Hydroxide | ad pH 5,7 |

**Rezepturbeispiel 19: "3 in 1" Care and Condtitioning Shampoo**

| Rohstoff | INCI | [%] |
|---|---|---|
| Wasser, demineralisiert | AQUA (WATER) | ad 100 |
| Ronastar® Noble Sparks | CALCIUM ALUMINUM BOROSILICATE, SILICA, CI 77891 (TITANIUM DIOXIDE), TIN OXIDE | 0,10 |
| Titandioxid aus Beispiel 3b | | 0,20 |
| Carbopol Aqua SF1 | ACRYLATES COPOLYMER | 8,00 |
| Texapon NSO | SODIUM LAURETH SULFATE | 40,00 |
| Natronlauge, 10 %ig | SODIUM HYDROXIDE | 0,00 |
| Tego Betain F 50 | COCAMIDOPROPYL BETAINE | 5,60 |
| ProtaFlor W25 | HYDROLYZED WHEAT PROTEIN, GLYCERIN, POLYQUATERNIUM-7 | 1,00 |
| Dow Corning 193 Fluid | PEG-12 DIMETHICONE | 3,00 |
| 0,1 % FD&C Yellow No. 5 in Wasser | AQUA (WATER), CI 19140 (FD&C YELLOW NO. 5) | 1,00 |
| Frag 280847 Vert & Pampelmousse | PARFUM | 0,40 |
| Brondinox L | Propylene Glycol, 5-BROMO-5-NITRO-1,3-DIOXANE | 0,30 |
| Oxynex ST Liquid | Diethylhexylsyringelydene Malonate, Carpric Caprylic Triglyceride | 0,10 |

**Rezepturbeispiel 20: Anti-Age Feuchtigkeitspflege Intensiv O/W**

| Ingredients | INCI | [%] |
|---|---|---|
| **A** | | |
| **Eusolex® OCR** | **OCTOCRYLENE** | **10.00** |
| **Eusolex® 9020** | **BUTYL METHOXYDIBENZOYLMETHANE** | **5.00** |
| Tego Care 450 | (2)POLYGLYCERYL-3 METHYLGLUCOSE DISTEARATE | 2.00 |
| Tegosoft TN | C12-15 ALKYL BENZOATE | 4.50 |
| Crodaderm S | SUCROSE POLYSOYATE | 2.00 |
| Syncrowax HGLC | C18-36 ACID TRIGLYCERIDE | 1.00 |
| Softisan 100 | HYDROGENATED COCO-GLYCERIDES | 1.00 |
| Antaron V-216 | PVP/HEXADECENE COPOLYMER | 1.00 |
| Shea butter | BUTYROSPERMUM PARKII (SHEA BUTTER) | 0.50 |
| Dow Corning 200 (100cs) | DIMETHICONE | 2.00 |
| Tween 20 | POLYSORBATE 20 | 0.25 |
| **RonaCare AP** | **Hydroxy Dimethoxybenzyl Malonate** | **0.20** |
| Propyl-4-hydroxybenzoate | PROPYLPARABEN | 0.05 |
| **B** | | |
| **Titandioxid aus Beispiel 3a** | **TITANIUM DIOXIDE**, **SILICA** | **1.00** |
| **RonaCare® Ectoin** | **ECTOIN** | **0.30** |
| 1,3-Butanediol | BUTYLENE GLYCOL | 5.00 |
| Glycerol, anhydrous | GLYCERIN | 1.75 |
| Titriplex® III | DISODIUM EDTA | 0.05 |
| Water, demineralized | AQUA (WATER) | 61.60 |
| Methyl-4-hydroxybenzoate | METHYLPARABEN | 0.15 |
| C | | |
| Pemulen TR-2 | ACRYLATES/C10-30 ALKYL ACRYLATE CROSSPOLYMER | 0.15 |
| Tegosoft TN Sodium hydroxide, | C12-15 ALKYL BENZOATE | 0.50 |
| 10% solution | SODIUM HYDROXIDE | q.s. |

(fortgesetzt)

| | | |
|---|---|---|
| C | | |
| Fragrance | PARFUM | q.s. |

**Rezepturbeispiel 21: Sonnenschutz extra hoch mit Radikalschutz O/W**

| Rohstoff | INCI | [%] |
|---|---|---|
| **A** | | |
| Titandioxid aus Beispiel 6b | | |
| Miglyol 8810 | BUTYLENE GLYCOL DICAPRYLATE/ DICAPRATE | 8,00 |
| Dehymuls PGPH | POLYGLYCERYL-2 DIPOLYHYDROXY-STEARATE | 1,00 |
| Eusolex® OCR | OCTOCRYLENE | 10,00 |
| Eusolex® 9020 | BUTYL METHOXYDIBENZOYLMETHANE | 4,00 |
| Eusolex® 4360 | BENZOPHENONE-3 | 4,00 |
| Emulgade F | SODIUM CETEARYL SULFATE, CETEARYL ALCOHOL, PEG-40 CASTOR OIL | 3,00 |
| Tegin | GLYCERYL STEARATE SE | 1,50 |
| Syncrowax HGLC | C18-36 ACID TRIGLYCERIDE | 1,50 |
| Softisan 100 | HYDROGENATED COCO-GLYCERIDES | 1,50 |
| Dow Corning 345 | CYCLOMETHICONE | 6,00 |
| X-Tend 226 | PHENYLETHYL BENZOATE | 4,50 |
| Antaron V-216 | PVP/HEXADECENE COPOLYMER | 1,00 |
| RonaCare AP | Hydroxy Dimethoxybenzyl Malonate | 0,50 |
| Propyl-4-hydroxybenzoat | PROPYLPARABEN | 0,05 |
| B | | |
| Eusolex® 232 | PHENYLBENZIMIDAZOLE SULFONIC ACID | 3,00 |
| Triethanolamin | TRIETHANOLAMINE | 1,60 |
| Keltrol RD | XANTHAN GUM | 0,20 |
| RonaCare® Ectoin | ECTOIN | 0,10 |
| Glycerin, wasserfrei | GLYCERIN | 5,00 |
| Titriplex® III | DISODIUM EDTA | 0,05 |
| Methyl-4-hydroxybenzoat | METHYLPARABEN | 0,15 |
| Wasser, demineralisiert | AQUA (WATER) | 39,35 |
| Parfümöl (q.s.) | PARFUM | 0,00 |

**Rezepturbeispiel 22: Feuchtigkeitssonnenmilch mit Zellschutz O/W**

| Rohstoff | INCI | [%] |
|---|---|---|
| **A** | | |
| Titandioxid aus Beispiel 6c | | 2,00 |
| Eusolex® OCR | OCTOCRYLENE | 9,00 |
| Eusolex® 9020 | BUTYL METHOXYDIBENZOYLMETHANE | 2,00 |
| Arlacel 165 VP | GLYCERYL STEARATE, PEG-100 STEARATE | 2,00 |
| Amphisol K | POTASSIUM CETYL PHOSPHATE | 1,50 |
| Lanette 16 | CETYL ALCOHOL | 1,00 |
| Stearinsäure | STEARIC ACID | 1,00 |
| Sheabutter fest | BUTYROSPERMUM PARKII (SHEA BUTTER) | 0,50 |
| Dow Corning 245 | CYCLOMETHICONE | 2,50 |

(fortgesetzt)

| Rohstoff | INCI | [%] |
|---|---|---|
| Arlamol HD | ISOHEXADECANE | 1,00 |
| Dow Corning 200 (10cs) | DIMETHICONE | 0,50 |
| Antaron V-216 | PVP/HEXADECENE COPOLYMER | 1,00 |
| Vitamin E-Acetat (DL-alpha-Tocopherolacetat)/Ph Eu | TOCOPHERYL ACETATE | 0,50 |
| **B** | | |
| Eusolex® UV-Pearls OMC | AQUA (WATER), ETHYLHEXYL METHOXYCINNAMATE, SILICA, PVP, CHLORPHENESIN, BHT | 3,00 |
| RonaCare® Ectoin | ECTOIN | 0,15 |
| Glycerin, wasserfrei | GLYCERIN | 5,00 |
| Propylenglykol, 1,2- | PROPYLENE GLYCOL | 4,00 |
| Keltrol CG-SFT | XANTHAN GUM | 0,10 |
| Wasser, demineralisiert | AQUA (WATER) | ad 100 |
| **C** | | |
| Pemulen TR-2 | ACRYLATES/C10-30 ALKYL ACRYLATE CROSSPOLYMER | 0,05 |
| Cetiol LC | COCO-CAPRYLATE/CAPRATE | 0,50 |
| Triethanolamin | TRIETHANOLAMINE | |
| Konservierungsmittel q.s. | PHENOXYETHANOL, ISOPROPYLPARABEN, ISOBUTYLPARABEN, BUTYLPARABEN | |
| Parfümöl (q.s.) | PARFUM | |

**Rezepturbeispiel 23: Sonnenmilch mit Hautschutzbalance O/W**

| Ingredients | INCI | [%] |
|---|---|---|
| **A** | | |
| **Titandioxid aus Beispiel 6a** | | **2,00** |
| Miglyol 8810 | BUTYLENE GLYCOL DICAPRYLATE/ DICAPRATE | 2,00 |
| Dehymuls PGPH | **POLYGLYCERYL-2DIPOLYHYDROXY-STEARATE** | **0,50** |
| **Eusolex® OCR** | **OCTOCRYLENE** | **4.50** |
| **Eusolex® 2292** | **ETHYLHEXYL METHOXYCINNAMATE, BHT** | **0.50** |
| **Eusolex® 9020** | **BUTYL METHOXYDIBENZOYLMETHANE** | **2.00** |
| Imwitor 372 P | GLYCERYL STEARATE CITRATE | 2.50 |
| Lanette 18 | STEARYL ALCOHOL | 1.50 |
| Softisan 100 | HYDROGENATED COCO-GLYCERIDES | 1.00 |
| Cetiol OE | DICAPRYLYL ETHER | 3.00 |
| Eutanol G | OCTYLDODECANOL | 3.00 |
| Dow Corning 345 | CYCLOMETHICONE | 2.50 |
| Antaron V-216 | PVP/HEXADECENE COPOLYMER | 0.50 |
| Vitamin E-Acetate | TOCOPHERYL ACETATE | 0.50 |
| Propyl-4-hydroxybenzoate | PROPYLPARABEN | 0.05 |
| **B** | | |
| Keltrol RD | XANTHAN GUM | 0.25 |
| **RonaCare® Ectoin** | **ECTOIN** | 0.10 |
| Glycerol, anhydrous | GLYCERIN | 5.00 |
| Titriplex® III | DISODIUM EDTA | 0.05 |
| Water, demineralized | AQUA (WATER) | ad 100 |
| Methyl-4-hydroxybenzoate | METHYLPARABEN | 0.15 |

(fortgesetzt)

| | | |
|---|---|---|
| C | | |
| Cetiol OE | DICAPRYLYL ETHER | 0.50 |
| Pemulen TR-2 | ACRYLATES/C10-30 ALKYL ACRYLATE CROSSPOLYMER | 0.10 |
| Triethanolamine | TRIETHANOLAMINE | 0.70 |
| Fragrance | PARFUM | q.s. |

Figur 1: In Figur 1 sind die Ergebnisse der continuous wave -GPR-Messungen gemäß Beispiel 10 (Methode 10a) wiedergegeben. Es wird die Spinkonzentration der jeweiligen Probe angegeben.

Figur 2: In Figur 2 sind die Ergebnisse des Wirksamkeitstests in vivo gegen photo-oxidativen Stress der Haut, induziert durch UVA-Bestrahlung wiedergegeben (Methode 10h). Es wird der Farb-Index nach UV-Bestrahlung der jeweiligen Probe angegeben.

Figur 3: In Figur 3 sind die Ergebnisse der Bestimmung der Produktfarbe in Formulierung für drei Formulierungen wiedergegeben (Methode 10c).

## Patentansprüche

1. UV-Schutzmittel, wobei der UV-Schutz im Wesentlichen von einem partikulären Titandioxid ausgeht, **dadurch gekennzeichnet, dass** das Titandioxid eine Mangan-haltige Beschichtung aufweist und dass die Kristallitgröße des partikulären Titandioxids in dem partikulären UV-Schutzmittel bestimmt nach der Scherrer-Methode im Bereich von 8 nm bis 50 nm liegt und die im Transmissionselektronenmikroskop ermittelbaren Abmessungen des partikulären Titandioxids bei einer Länge von 20 bis 60 nm und einer Breite von 8 bis 30 nm liegen.

2. UV-Schutzmittel nach Anspruch 1, **dadurch gekennzeichnet, dass** es erhältlich ist durch hydrothermale Behandlung eines partikulären Titandioxids und anschließendes Aufbringen mindestens einer Beschichtung.

3. UV-Schutzmittel nach Anspruch 1 oder 2, **dadurch gekennzeichnet, dass** es sich bei dem Titandioxid im Wesentlichen um Titandioxid handelt, das mit Metallionen, wie insbesondere Eisen- oder Cer-Ionen, dotiert ist.

4. UV-Schutzmittel nach einem oder mehreren der Ansprüche 1 bis 3, **dadurch gekennzeichnet, dass** der Titandioxid-Grundkörper eine erste Beschichtung im Wesentlichen bestehend aus Mangan-Verbindungen trägt und eine zweite Beschichtung im Wesentlichen bestehend aus Aluminium und/oder Siliciumverbindungen trägt.

5. UV-Schutzmittel nach einem oder mehreren der Ansprüche 1 bis 3, **dadurch gekennzeichnet, dass** der Titandioxid-Grundkörper eine erste Beschichtung im Wesentlichen bestehend aus Aluminium und/oder Siliciumverbindungen trägt und eine zweite Beschichtung im Wesentlichen bestehend aus Mangan-Verbindungen trägt.

6. UV-Schutzmittel nach einem oder mehreren der Ansprüche 1 bis 3, **dadurch gekennzeichnet, dass** der Titandioxid-Grundkörper eine Beschichtung im Wesentlichen bestehend aus einem Gemisch von Mangan-Verbindungen mit Aluminium und/oder Siliciumverbindungen trägt.

7. UV-Schutzmittel nach einem oder mehreren der Ansprüche 1 bis 6, **dadurch gekennzeichnet, dass** die Mangan-Verbindung(en) ausgewählt sind aus den Oxiden, Hydroxiden, Phosphaten, Sulfaten und Fettsäuresalzen des Mangans.

8. UV-Schutzmittel nach einem oder mehreren der Ansprüche 1 bis 7, **dadurch gekennzeichnet, dass** die äußere Schicht eine hydrophobierende Schicht ist.

9. UV-Schutzmittel nach einem oder mehreren der Ansprüche 4 bis 8, **dadurch gekennzeichnet, dass** die gesamte Beschichtung des Titandioxids bezogen auf das gesamte partikuläre UV-Schutzmittel 5 Gew.% bis 50 Gew.% beträgt, wobei der Anteil der Manganhaltigen Schicht bezogen auf das gesamte partikuläre UV-Schutzmittel 0,1 Gew.% bis 1 Gew.% beträgt.

**10.** Verfahren zur Herstellung eines partikulären UV-Schutzmittels nach Anspruch 1, **dadurch gekennzeichnet, dass**

a) ein partikuläres Titandioxid hydrothermal behandelt wird und
b) anschließend eine Mangan-haltige-Beschichtung aufgebracht wird.

**11.** Verfahren nach Anspruch 10, **dadurch gekennzeichnet, dass** Schritt a) in einem geschlossenen Behälter bei Temperaturen im Bereich von 40 bis 360°C durchgeführt wird.

**12.** Verfahren nach Anspruch 10 oder 11, **dadurch gekennzeichnet, dass** Schritt b) als Sol-Gel-Prozess durchgeführt wird, wobei vorzugsweise eine Mangansulfat-Lösung zu einer Suspension des Titandioxids gegeben wird.

**13.** Verfahren nach einem oder mehreren der Ansprüche 10 bis 12, **dadurch gekennzeichnet, dass** Schritt b) im Bereich von pH = 2 bis pH = 11 erfolgt.

**14.** Verfahren nach einem oder mehreren der Ansprüche 10 bis 13, **dadurch gekennzeichnet, dass** Schritt b) bei einer Temperatur im Bereich von 50°C bis 100°C durchgeführt wird.

**15.** Verfahren nach einem oder mehreren der Ansprüche 10 bis 14, **dadurch gekennzeichnet, dass** in einer Nachbehandlung eine hydrophobierende Schicht aufgebracht wird.

**16.** Wässrige oder ölige Dispersion, enthaltend ein partikuläres UV-Schutzmittel nach einem oder mehreren der Ansprüche 1 bis 9.

**17.** Zubereitung mit Lichtschutzeigenschaften enthaltend mindestens ein UV-Schutzmittel nach einem oder mehreren der Ansprüche 1 bis 9 oder ein partikuläres Titandioxid, hergestellt nach einem Verfahren entsprechend mindestens einem der Ansprüche 10 bis 15.

**18.** Zubereitung mit Lichtschutzeigenschaften nach Anspruch 17, **dadurch gekennzeichnet, dass** es sich um eine topisch anwendbare Zubereitung, vorzugsweise eine kosmetische oder dermatologische Formulierung, handelt.

**19.** Zubereitung mit Lichtschutzeigenschaften nach Anspruch 17 oder 18, **dadurch gekennzeichnet, dass** die Zubereitung mindestens einen organischen UV-Filter enthält.

**20.** Zubereitung mit Lichtschutzeigenschaften nach Anspruch 19, **dadurch gekennzeichnet, dass** die Zubereitung ein Dibenzoylmethanderivat und/oder ein Benzophenon-Derivat enthält.

**21.** Zubereitung mit Lichtschutzeigenschaften nach einem oder mehreren der Ansprüche 17 bis 20, **dadurch gekennzeichnet, dass** die Zubereitung mindestens einen Selbstbräuner enthält.

**22.** Zubereitung mit Lichtschutzeigenschaften nach einem oder mehreren der Ansprüche 17 bis 21, **dadurch gekennzeichnet, dass** die Zubereitung mindestens einen Photostabilisator, vorzugsweise entsprechend der Formel IV

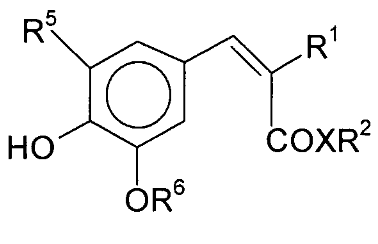

IV,

wobei
$R^1$ ausgewählt ist aus $-C(O)CH_3$, $-CO_2R^3$, $-C(O)NH_2$ and $-C(O)N(R^4)_2$; X is O or NH;
$R^2$ steht für einen linearen oder verzweigten $C_{1-30}$-Alkylrest;
$R^3$ steht für einen linearen oder verzweigten $C_{1-20}$-Alkylrest,
alle $R^4$ unabhängig voneinander stehen für H oder lineare oder verzweigte $C_{1-8}$-Alkylreste,
$R^5$ steht für H, einen linearen oder verzweigten $C_{1-8}$-Alkylrest oder einen linearen oder verzweigten $-O-C_{1-8}$-Alkylrest und

$R^6$ steht für einen $C_{1-8}$-Alkylrest,
enthält.

**23.** Zubereitung mit Lichtschutzeigenschaften nach einem oder mehreren der Ansprüche 17 bis 22, **dadurch gekennzeichnet, dass** die Zubereitung einen oder mehrere weitere UV-Filter enthält, die ausgewählt sind aus der Gruppe 3-(4'-Methylbenzyliden)-dl-kampfer, Methoxyzimtsäureoctylester, 3,3,5-Trimethyl-cyclohexylsalicylat, 4-(Dimethylamino)benzoesäure2-ethylhexylester, 2-Cyano-3,3-diphenyl-acrylsäure-2-ethylhexylester, 2-Phenylbenzimidazol-5-sulfonsäure sowie ihre Kalium-, Natrium- und Triethanolaminsalze.

**24.** Zubereitung mit Lichtschutzeigenschaften nach einem oder mehreren der Ansprüche 17 bis 23 geeignet zum Schutz von Körperzellen gegen oxidativen Stress, **dadurch gekennzeichnet, dass** sie ein oder mehrere Antioxidantien enthält.

**25.** Zubereitung mit Lichtschutzeigenschaften nach einem oder mehreren der Ansprüche 17 bis 24, **dadurch gekennzeichnet, dass** es sich um eine Emulgator-freie Emulsion handelt.

**26.** Verfahren zur Herstellung einer Zubereitung nach einem oder mehreren der Ansprüche 17 bis 25, **dadurch gekennzeichnet, dass** ein partikuläres UV-Schutzmittel nach einem oder mehreren der Ansprüche 1 bis 9 oder ein partikuläres UV-Schutzmittel, hergestellt nach einem oder mehreren der Ansprüche 10 bis 15, mit einem kosmetisch oder dermatologisch geeignetem Träger und ggf. weiteren Inhaltstoffen vermischt wird.

**27.** Verwendung eines partikulären UV-Schutzmittels nach einem oder mehreren der Ansprüche 1 bis 9 oder eines partikulären UV-Schutzmittels, hergestellt nach einem oder mehreren der Ansprüche 10 bis 15, zur Stabilisierung von Selbstbräunern.

## Claims

**1.** UV protection agent, where the UV protection essentially emanates from a particulate titanium dioxide, **characterised in that** the titanium dioxide has a manganese-containing coating and **in that** the crystallite size of the particulate titanium dioxide in the particulate UV protection agent, determined by the Scherrer method, is in the range from 8 nm to 50 nm, and the dimensions of the particulate titanium dioxide, which can be determined in a transmission electron microscope, are a length of 20 to 60 nm and a width of 8 to 30 nm.

**2.** UV protection agent according to Claim 1, **characterised in that** it is obtainable by hydrothermal treatment of a particulate titanium dioxide and subsequent application of at least one coating.

**3.** UV protection agent according to Claim 1 or 2, **characterised in that** the titanium dioxide is essentially titanium dioxide which has been doped with metal ions, such as, in particular, iron or cerium ions.

**4.** UV protection agent according to one or more of Claims 1 to 3, **characterised in that** the titanium dioxide substrate carries a first coating essentially consisting of manganese compounds and a second coating essentially consisting of aluminium and/or silicon compounds.

**5.** UV protection agent according to one or more of Claims 1 to 3, **characterised in that** the titanium dioxide substrate carries a first coating essentially consisting of aluminium and/or silicon compounds and a second coating essentially consisting of manganese compounds.

**6.** UV protection agent according to one or more of Claims 1 to 3, **characterised in that** the titanium dioxide substrate carries a coating essentially consisting of a mixture of manganese compounds with aluminium and/or silicon compounds.

**7.** UV protection agent according to one or more of Claims 1 to 6, **characterised in that** the manganese compound(s) is (are) selected from the oxides, hydroxides, phosphates, sulfates and fatty acid salts of manganese.

**8.** UV protection agent according to one or more of Claims 1 to 7, **characterised in that** the outer layer is a hydrophobicising layer.

**9.** UV protection agent according to one or more of Claims 4 to 8, **characterised in that** the entire coating of the titanium dioxide, based on the entire particulate UV protection agent, is 5% by weight to 50% by weight, where the proportion of the manganese-containing layer, based on the entire particulate UV protection agent, is 0.1 % by weight to 1 % by weight.

**10.** Process for the preparation of a particulate UV protection agent according to Claim 1, **characterised in that**

a) a particulate titanium dioxide is subjected to hydrothermal treatment and
b) a manganese-containing coating is subsequently applied.

**11.** Process according to Claim 10, **characterised in that** step a) is carried out in a closed container at temperatures in the range from 40 to 360°C.

**12.** Process according to Claim 10 or 11, **characterised in that** step b) is carried out as a sol-gel process, in which a manganese sulfate solution is preferably added to a suspension of the titanium dioxide.

**13.** Process according to one or more of Claims 10 to 12, **characterised in that** step b) is carried out in the range from pH = 2 to pH = 11.

**14.** Process according to one or more of Claims 10 to 13, **characterised in that** step b) is carried out at a temperature in the range from 50°C to 100°C.

**15.** Process according to one or more of Claims 10 to 14, **characterised in that** a hydrophobicising layer is applied in a post-treatment.

**16.** Aqueous or oily dispersion comprising a particulate UV protection agent according to one or more of Claims 1 to 9.

**17.** Preparation having light-protection properties comprising at least one UV protection agent according to one or more of Claims 1 to 9 or a particulate titanium dioxide prepared by a process corresponding to at least one of Claims 10 to 15.

**18.** Preparation having light-protection properties according to Claim 17, **characterised in that** it is a preparation which can be applied topically, preferably a cosmetic or dermatological formulation.

**19.** Preparation having light-protection properties according to Claim 17 or 18, **characterised in that** the preparation comprises at least one organic UV filter.

**20.** Preparation having light-protection properties according to Claim 19, **characterised in that** the preparation comprises a dibenzoylmethane derivative and/or a benzophenone derivative.

**21.** Preparation having light-protection properties according to one or more of Claims 17 to 20, **characterised in that** the preparation comprises at least one self-tanning agent.

**22.** Preparation having light-protection properties according to one or more of Claims 17 to 21, **characterised in that** the preparation comprises at least one photostabiliser, preferably conforming to the formula IV

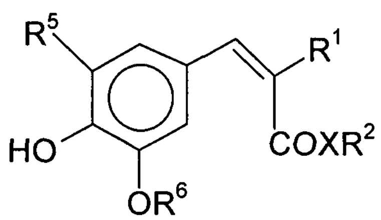

IV,

where

$R^1$ is selected from $-C(O)CH_3$, $-CO_2R^3$, $-C(O)NH_2$ and $-C(O)N(R^4)_2$;
X is O or NH;

$R^2$ stands for a linear or branched $C_{1-30}$-alkyl radical;
$R^3$ stands for a linear or branched $C_{1-20}$-alkyl radical,
all $R^4$, independently of one another, stand for H or linear or branched $C_{1-8}$-alkyl radicals,
$R^5$ stands for H, a linear or branched $C_{1-8}$-alkyl radical or a linear or branched -O-$C_{1-8}$-alkyl radical and
$R^6$ stands for a $C_{1-8}$-alkyl radical.

**23.** Preparation having light-protection properties according to one or more of Claims 17 to 22, **characterised in that** the preparation comprises one or more further UV filters which are selected from the group 3-(4'-methylbenzylidene)-dl-camphor, octyl methoxycinnamate, 3,3,5-trimethylcyclohexyl salicylate, 2-ethylhexyl 4-(dimethylamino)-benzoate, 2-ethylhexyl 2-cyano-3,3-diphenylacrylate, 2-phenyl-benzimidazole-5-sulfonic acid and the potassium, sodium and triethanolamine salts thereof.

**24.** Preparation having light-protection properties according to one or more of Claims 17 to 23 suitable for the protection of body cells against oxidative stress, **characterised in that** it comprises one or more antioxidants.

**25.** Preparation having light-protection properties according to one or more of Claims 17 to 24, **characterised in that** it is an emulsifier-free emulsion.

**26.** Process for the preparation of a preparation according to one or more of Claims 17 to 25, **characterised in that** a particulate UV protection agent according to one or more of Claims 1 to 9 or a particulate UV protection agent prepared according to one or more of Claims 10 to 15 is mixed with a cosmetically or dermatologically suitable vehicle and optionally further ingredients.

**27.** Use of a particulate UV protection agent according to one or more of Claims 1 to 9 or a particulate UV protection agent prepared according to one or more of Claims 10 to 15 for the stabilisation of self-tanning agents.

## Revendications

**1.** Agent de protection anti-UV, où la protection anti-UV a essentiellement pour origine un dioxyde de titane particulaire, **caractérisé en ce que** le dioxyde de titane possède un revêtement contenant du manganèse et **en ce que** la taille de cristallite du dioxyde de titane particulaire dans l'agent de protection anti-UV particulaire , déterminée par la méthode de Scherrer, se trouve dans la plage allant de 8 nm à 50 nm, et les dimensions du dioxyde de titane particulaire, qui peuvent être déterminées à l'aide d'un microscope électronique en transmission, sont d'une longueur allant de 20 à 60 nm et d'une largeur allant de 8 à 30 nm.

**2.** Agent de protection anti-UV selon la revendication 1, **caractérisé en ce qu'**il peut être obtenu par traitement hydrothermal d'un dioxyde de titane particulaire et application ultérieure d'au moins un revêtement.

**3.** Agent de protection anti-UV selon la revendication 1 ou 2, **caractérisé en ce que** le dioxyde de titane est essentiellement du dioxyde de titane ayant été dopé par des ions métalliques, tels que, en particulier, des ions fer ou cérium.

**4.** Agent de protection anti-UV selon l'une ou plusieurs parmi les revendications 1 à 3, **caractérisé en ce que** le substrat de dioxyde de titane porte un premier revêtement constitué essentiellement de composés de manganèse et un deuxième revêtement constitué essentiellement de composés d'aluminium et/ou de silicium.

**5.** Agent de protection anti-UV selon l'une ou plusieurs parmi les revendications 1 à 3, **caractérisé en ce que** le substrat de dioxyde de titane porte un premier revêtement constitué essentiellement de composés d'aluminium et/ou de silicium et un deuxième revêtement constitué essentiellement de composés de manganèse.

**6.** Agent de protection anti-UV selon l'une ou plusieurs parmi les revendications 1 à 3, **caractérisé en ce que** le substrat de dioxyde de titane porte un revêtement constitué essentiellement d'un mélange de composés de manganèse avec des composés d'aluminium et/ou de silicium.

**7.** Agent de protection anti-UV selon l'une ou plusieurs parmi les revendications 1 à 6, **caractérisé en ce que** le ou les composés de manganèse sont choisis parmi les oxydes, hydroxydes, phosphates, sulfates et sels d'acides gras de manganèse.

8. Agent de protection anti-UV selon l'une ou plusieurs parmi les revendications 1 à 7, **caractérisé en ce que** la couche externe est une couche rendant hydrophobe.

9. Agent de protection anti-UV selon l'une ou plusieurs parmi les revendications 4 à 8, **caractérisé en ce que** l'ensemble du revêtement de dioxyde de titane, sur la base de l'ensemble de l'agent de protection anti-UV particulaire, va de 5% en poids à 50% en poids, où la proportion de la couche contenant du manganèse, sur la base de l'ensemble de l'agent de protection anti-UV particulaire, va de 0,1% en poids à 1% en poids.

10. Procédé de préparation d'un agent de protection anti-UV particulaire selon la revendication 1, **caractérisé en ce que**

    a) un dioxyde de titane particulaire est soumis à un traitement hydrothermal et
    b) un revêtement contenant du manganèse est ultérieurement appliqué.

11. Procédé selon la revendication 10, **caractérisé en ce que** l'étape a) est effectuée dans un conteneur fermé à des températures dans la plage allant de 40 à 360°C.

12. Procédé selon la revendication 10 ou 11, **caractérisé en ce que** l'étape b) est effectuée sous forme de procédé sol-gel, dans lequel une solution de sulfate de manganèse est ajoutée de préférence à une suspension du dioxyde de titane.

13. Procédé selon l'une ou plusieurs parmi les revendications 10 à 12, **caractérisé en ce que** l'étape b) est effectuée dans une plage allant de pH = 2 à pH = 11.

14. Procédé selon l'une ou plusieurs parmi les revendications 10 à 13, **caractérisé en ce que** l'étape b) est effectuée à une température dans la plage allant de 50°C à 100°C.

15. Procédé selon l'une ou plusieurs parmi les revendications 10 à 14, **caractérisé en ce qu'**une couche rendant hydrophobe est appliquée dans un post-traitement.

16. Dispersion aqueuse ou huileuse comprenant un agent de protection anti-UV particulaire selon l'une ou plusieurs parmi les revendications 1 à 9.

17. Préparation ayant des propriétés de photo-protection, comprenant au moins un agent de protection anti-UV selon l'une ou plusieurs parmi les revendications 1 à 9 ou un dioxyde de titane particulaire préparé par un procédé correspondant à au moins l'une des revendications 10 à 15.

18. Préparation ayant des propriétés de photo-protection selon la revendication 17, **caractérisée en ce qu'**il s'agit d'une préparation pouvant être appliquée par voie topique, préférablement une formulation cosmétique ou dermatologique.

19. Préparation ayant des propriétés de photo-protection selon la revendication 17 ou 18, **caractérisée en ce que** la préparation comprend au moins un filtre anti-UV organique.

20. Préparation ayant des propriétés de photo-protection selon la revendication 19, **caractérisée en ce que** la préparation comprend un dérivé de dibenzoylméthane et/ou un dérivé de benzophénone.

21. Préparation ayant des propriétés de photo-protection selon l'une ou plusieurs parmi les revendications 17 à 20, **caractérisée en ce que** la préparation comprend au moins un agent auto-bronzant.

22. Préparation ayant des propriétés de photo-protection selon l'une ou plusieurs parmi les revendications 17 à 21, **caractérisée en ce que** la préparation comprend au moins un photostabilisant, répondant de préférence à la formule IV

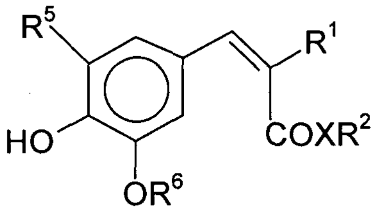

IV,

dans laquelle

$R^1$ est choisi parmi $-C(O)CH_3$, $-CO_2R^3$, $-C(O)NH_2$ et $-C(O)N(R^4)_2$ ; X est O ou NH ;
$R^2$ représente un radical $C_{1-30}$-alkyle linéaire ou ramifié ;
$R^3$ représente un radical $C_{1-20}$-alkyle linéaire ou ramifié ;
tous les $R^4$, indépendamment les uns des autres, représentent H ou des radicaux $C_{1-8}$-alkyle linéaires ou ramifiés ;
$R^5$ représente H, un radical $C_{1-8}$-alkyle linéaire ou ramifié ou un radical $-O-C_{1-8}$-alkyle linéaire ou ramifié ; et
$R^6$ représente un radical $C_{1-8}$-alkyle.

**23.** Préparation ayant des propriétés de photo-protection selon l'une ou plusieurs parmi les revendications 17 à 22, **caractérisée en ce que** la préparation comprend un ou plusieurs autres filtres anti-UV qui sont choisis dans le groupe constitué par le 3-(4'-méthylbenzylidène)-dl-camphre, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phényl-benzimidazole-5-sulfonique et les sels de potassium, sodium et triéthanolamine de celui-ci.

**24.** Préparation ayant des propriétés de photo-protection selon l'une ou plusieurs parmi les revendications 17 à 23, convenable pour la protection de cellules de l'organisme contre un stress oxydatif, **caractérisée en ce qu'**elle comprend un ou plusieurs antioxydants.

**25.** Préparation ayant des propriétés de photo-protection selon l'une ou plusieurs parmi les revendications 17 à 24, **caractérisée en ce qu'**il s'agit d'une émulsion dépourvue d'émulsifiants.

**26.** Procédé de préparation d'une préparation selon l'une ou plusieurs parmi les revendications 17 à 25, **caractérisé en ce qu'**un agent de protection anti-UV particulaire selon l'une ou plusieurs parmi les revendications 1 à 9 ou un agent de protection anti-UV particulaire préparé selon l'une ou plusieurs parmi les revendications 10 à 15, est mélangé avec un véhicule convenable sur le plan cosmétique ou dermatologique et éventuellement d'autres ingrédients.

**27.** Utilisation d'un agent de protection anti-UV particulaire selon l'une ou plusieurs parmi les revendications 1 à 9 ou d'un agent de protection anti-UV particulaire préparé selon l'une ou plusieurs parmi les revendications 10 à 15, pour la stabilisation d'agents auto-bronzants.

Fig. 1

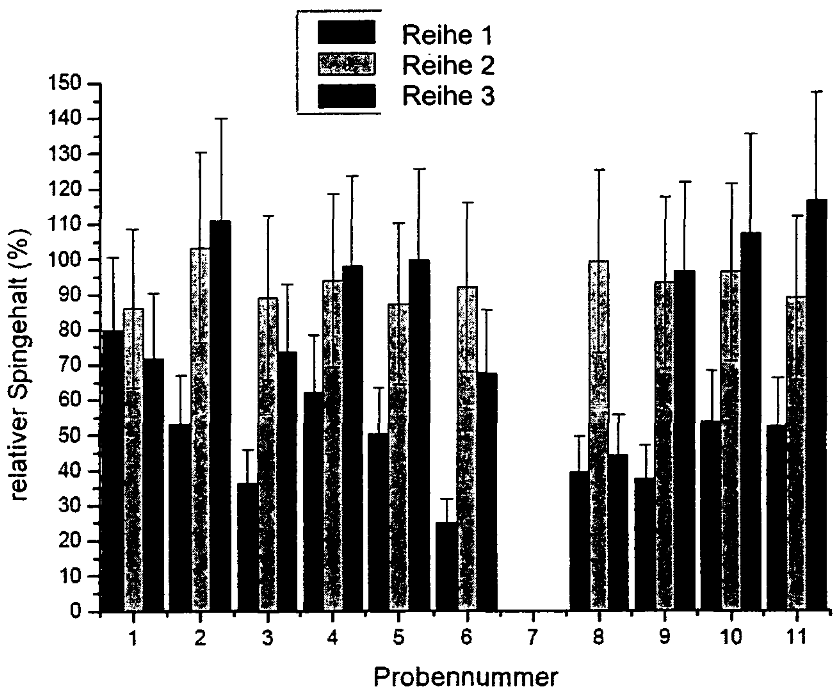
Reihe 1
Reihe 2
Reihe 3
relativer Spingehalt (%)
0
10
20
30
40
50
60
70
80
90
100
110
120
130
140
150
1
2
3
4
5
6
7
8
9
10
11
Probennummer

Fig. 2

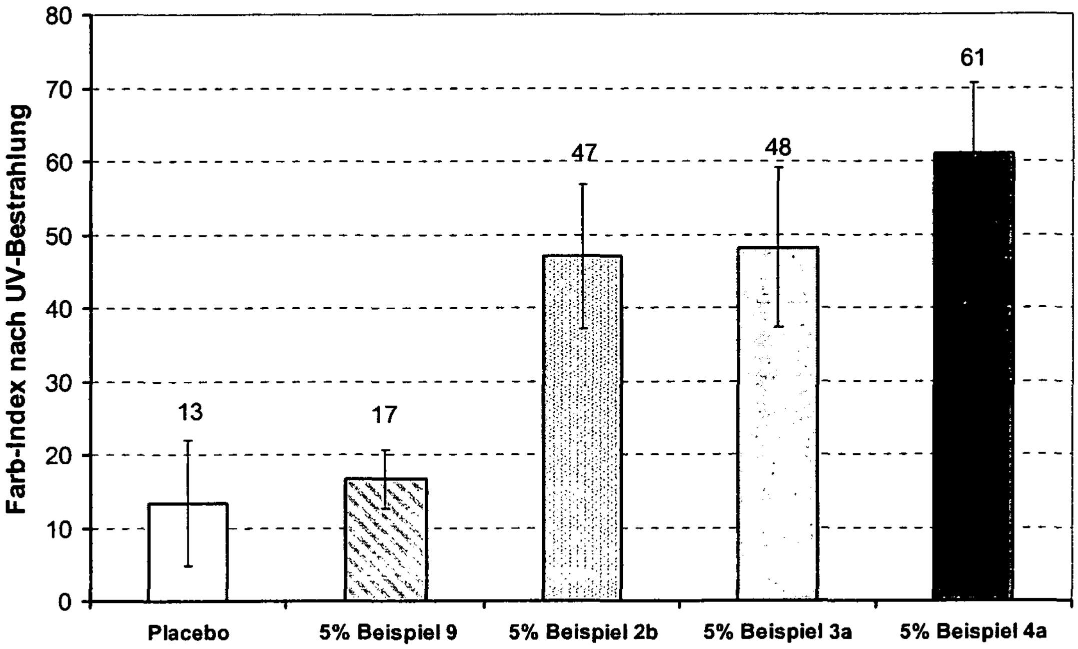
Farb-Index nach UV-Bestrahlung
80
70
60
50
40
30
20
10
0
13
17
47
48
61
Placebo
5% Beispiel 9
5% Beispiel 2b
5% Beispiel 3a
5% Beispiel 4a

Fig. 3

Beispiel 3a Beispiel 3b Beispiel 7

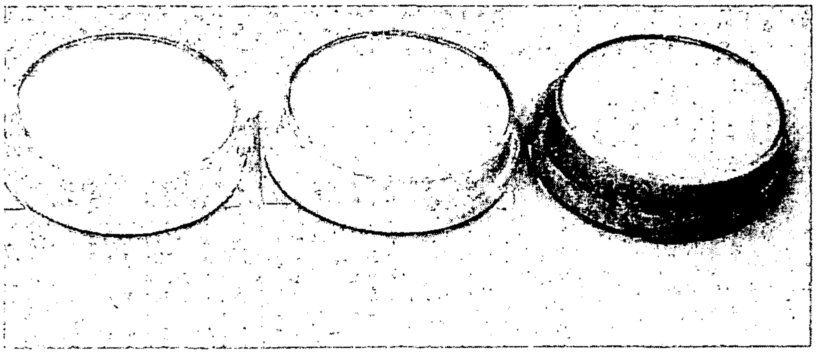

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- FR 2326405 A
- FR 2440933 A
- EP 0114607 A
- WO 9960994 A
- US 5643592 A
- DE 19826379
- GB 1222955 A
- GB 1162799 A
- WO 2007141342 A
- WO 2008015056 A
- WO 2007065574 A
- WO 9304665 A
- EP 0487404 A
- WO 9966896 A
- US 6242099 B1
- WO 0009652 A
- WO 0072806 A
- WO 0071084 A
- WO 2006111233 A
- EP 0671161 A
- DE 4116123 A
- WO 03007906 A
- DE 4308282 A


### In der Beschreibung aufgeführte Nicht-Patentliteratur


- Einkristallzüchtung. **K.RECKER.** Ullmanns Enzyklopädie der Technischen Chemie. 1978, vol. 15, 117 ff
- *Cosmetics & Toiletries,* Februar 1990, vol. 105, 53-64
- Rowe Colour Index. Society of Dyers and Colourists, 1971
- **E. A. GALINSKI et al.** *Eur. J. Biochem.,* 1985, vol. 149, 135-139
- **CHAN, S.Y. ; LI WAN PO, A.** Quantitative evaluation of drug-induced erythema by using a tristimulus colour analyzer: experimental design and data analysis. *Skin. Pharmacol,* 1993, vol. 6, 298-312
- **ECKHARDT, L. ; MAYER, J.A. ; CREECH, L. ; JOHNSTON, M.R. ; LUI, K.J. ; SALLIS, J.F. ; ELDER, J.P.** Assessing children's ultraviolet radiation exposure: the potential usefulness of a colorimeter. *Am. J. Public Health,* 1996, vol. 86, 1802-1804
- **FLUHR, J.W. ; PFISTERER, S. ; GLOOR, M.** Direct comparison of skin physiology in children and adults with bioengineering methods. *Pediatr. Dermatol.,* 2000, vol. 17, 436-439
- **FULLERTON, A. ; BENEFELDT, E. ; PETERSEN, J.R. ; JENSEN, S.B. ; SERUP, J.** The calcipotrion dose-irritation relationship: 48 hour occlusive testing in healthy volunteers using Finn Chambers. *Br. J. Dermatol.,* 1998, vol. 138, 259-265
- **GARIGUE, J. ; MARGUERY, M.C. ; MALMARY, M.F. ; EL SAYED, F. ; BAZEX, J.** Measurement of actinic erythema in healthy subjects and in subjects with polymorphous light eruption using a tristimulus colorimeter. *Dermatology,* 1995, vol. 190, 31-34
- **GASSMUELLER, J. ; MAAS-IRSLINGER, R. ; RIPPKE, F. ; TAUSCH, I.** Antiinflammatorische Wirksamkeit magistraler Rezepturen mit Glukokortikosteroiden in Eucerinum-Fertiggrundlagen im Vergleich zu Fertigpräparaten im UVB-Erythemtest. Zeitschrift für Hautkrankheiten. *H+G,* 1998, vol. 6, 364-370
- **GUARRERA, M. ; BRUSATI, C. ; REBORA, A.** Topical metronidazole does not abate uvb-induced erythema. *Dermatology,* 2001, vol. 203, 121-123
- **HENRY, F. ; FUMAL, I. ; PIERARD, G.E.** Postural skin colour changes during the corticosteroid blanching assay. *Skin. Pharmacol. Appl. Skin. Physiol.,* 1999, vol. 12, 199-210
- **SEITZ, J.C. ; WHITMORE, C.G.** Measurement of erythema and tanning responses in human skin using a tristimulus colorimeter. *Dermatologica,* 1988, vol. 177, 70-75
- **TRONNIER, M. ; SCHULZ, R. ; WOLFF, H.H.** Colorimetrische Erythemmessung nach UVB-Bestrahlung an gesunder Haut in Abhängigkeit von unterschiedlicher Vorbehandlung. *Akt. Dermatol.,* 1992, vol. 18, 183-186